(19) Europäisches Patentamt / European Patent Office / Office européen des brevets

(11) EP 3 414 216 B1

(12) # EUROPEAN PATENT SPECIFICATION

(45) Date of publication and mention of the grant of the patent:
**21.10.2020 Bulletin 2020/43**

(21) Application number: **17706674.3**

(22) Date of filing: **06.02.2017**

(51) Int Cl.:
*C07B 43/04* (2006.01) *C07C 209/10* (2006.01)
*C07C 213/02* (2006.01) *C07D 265/30* (2006.01)
*C07D 295/033* (2006.01) *C07D 413/10* (2006.01)
*C07D 209/08* (2006.01) *C07D 217/02* (2006.01)
*C07D 295/073* (2006.01) *C07D 295/096* (2006.01)
*C07D 333/70* (2006.01) *C07D 491/113* (2006.01)

(86) International application number:
**PCT/DK2017/050025**

(87) International publication number:
**WO 2017/137047 (17.08.2017 Gazette 2017/33)**

(54) **ARYLATION OF ALIPHATIC AMINES**
ARYLIERUNG VON ALIPHATISCHEN AMINEN
ARYLATION D'AMINES ALIPHATIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA**

(30) Priority: **08.02.2016 DK 201670066**

(43) Date of publication of application:
**19.12.2018 Bulletin 2018/51**

(73) Proprietor: **AAA Chemistry ApS**
**3650 Ølstykke (DK)**

(72) Inventors:
- **DINESS, Frederik**
  **3650 Ølstykke (DK)**
- **MELDAL, Morten**
  **2400 Copenhagen NV (DK)**
- **JACOBSEN, Christian Borch**
  **3400 Hillerød (DK)**

(74) Representative: **Høiberg P/S**
**Adelgade 12**
**1304 Copenhagen K (DK)**

(56) References cited:

- **LOUIE J ET AL: "Palladium-Catalyzed Synthesis of Arylamines from Aryl Halides. Mechanistic Studies Lead to Coupling in the Absence of Tin Reagents", TETRAHEDRON LETTERS, ELSEVIER, AMSTERDAM, NL, vol. 36, no. 21, 22 May 1995 (1995-05-22), pages 3609-3612, XP004028031, ISSN: 0040-4039, DOI: 10.1016/0040-4039(95)00605-C**
- **FREDERIK DINESS ET AL: "Catalyst-Free N-Arylation Using Unactivated Fluorobenzenes", ANGEWANDTE CHEMIE INTERNATIONAL EDITION, vol. 51, no. 32, 6 August 2012 (2012-08-06), pages 8012-8016, XP55365079, ISSN: 1433-7851, DOI: 10.1002/anie.201202149**
- **SHELKAR RADHESHYAM S ET AL: "Ligand-free C-C and C-N cross-couplings with Pd/Nf-G nanoc", TETRAHEDRON LETTERS, vol. 56, no. 30, 2015, pages 4463-4467, XP029180749, ISSN: 0040-4039, DOI: 10.1016/J.TETLET.2015.05.091**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Technical field

The disclosure relates to a method for arylation of amines, such as aliphatic amines by reaction of aryl-halogens, e.g. chloro- or fluorobenzene derivatives without strongly electron withdrawing substituents in the presence of a strong base.

### Background

*N*-arylations of aliphatic amines are important chemical transformations as the resulting aniline derivatives have found broad applications as pharmaceuticals, materials for organic electronics and dyes for industrial and research applications. Pyrazine and morpholine derivatives are of special interest, as these are found in a range of top selling pharmaceuticals.

A range of transition metal-catalyzed reactions has been developed for the formal halide to nitrogen substitution on aryl halides. Most renowned are the Ullmann and Buchwald-Hartwig couplings employing copper and palladium catalysis respectively. These results notwithstanding, the employment of transition metals as catalysts has several drawbacks in industrial applications, especially due to high costs, oxygen sensitivity, challenging purifications and toxic metal contaminants being present in the final products.

To overcome these shortcomings, catalyst-free $S_NAr$ reactions on highly activated halide-substituted benzene derivatives have been applied. However, the scope of this approach has so far been limited as strongly electron-withdrawing groups, such as nitro or cyano substituents, have been considered essential for reactivity. Thus, leading text books in organic chemistry describes: a) "Without electron-attracting groups present, nucleophilic aromatic substitution occurs only under extreme reaction conditions" F. A. Carey, R. J. Sundberg in Advanced Organic Chemistry: Part A: Structure and Mechanisms; 4th ed. Springer Science and Business Media, New York, 2000. b) "To summarize: Any anion-stabilizing (electron-withdrawing) group ortho or para to a potential leaving group can be used to make nucleophilic aromatic substitution possible." J. Clayden, N. Greeves, S. Warren, P. Wothers in Organic Chemistry; Oxford University Press, New York, 2001.

WO 2014/191548 discloses a synthetic process for the production of 1-(2-((2,4-dimethyl-phenyl)sulfonyl)phenyl)piperazine by arylation in the presence of $Cs_2CO_3$. The process however requires incubation at elevated temperature for more than 14 days.

Louie, J. et al., Palladium-catalyzed Synthesis of Arylamines from Aryl Halides. Mechanistic Studies Lead to Coupling in the Absence of Tin Reagents, Tetrahedron Letters 1995, 36, 21, 3609-3612 discloses amination reactions of p-Bu-bromobenzene with piperidine, with LiHMDS as base catalysed by Pd. The method however requires catalysation by Pd.

Diness, F. et al., Catalyst-Free N-Arylation Using Unactivated Fluorobenzenes, Angewandte Chemie Int. Ed. 2012, 51, 32, 8012-8016 discloses a method for N-arylation of azoles and indoles from unactivated monofluorobenzenes.

### Summary

The present disclosure provides catalyst free N-arylation of amines. The methods are very effective, and can typically results in high yields.

Thus, the disclosure provides methods for preparing an arylated amine, said method comprising the steps of

a. Providing a nucleophile, wherein said nucleophile comprises an -NH- or an $-NH_2$ group directly linked to only non-aromatic carbon atoms or a salt of said nucleophile;

b. Providing an electrophile, wherein said electrophile is aryl substituted with at least two substituents, wherein the first substituent is halogen and the second substituent and any further optional substituent(s) are selected from the group consisting of halogen, aryl, substituted aryl, alkenyl, substituted alkenyl, heteroalkenyl, alkyl, substituted alkyl, heteroalkyl, alkoxy, substituted alkoxy, amino, substituted amino, thioalkyl, substituted thioalkyl, thioaryl, substituted thioaryl, heteroaryl, and phosphinyl, with the proviso that said aryl is substituted with at the most 4 halogens;

c. Providing a base, wherein the corresponding acid has a pKa above 32 in DMSO and/or a pKa above 26 in THF;

d. Providing an organic solvent that only contain protons with a pKa above 32 in DMSO.

e. Reacting said nucleophile with said electrophile in said organic solvent in the presence of the base, thereby obtaining an arylated amine consisting of said aryl, wherein the first substituent is substituted by said amine;

f. Optionally purifiying the arylated amine.

## Description of Drawings

Figure 1: Examples of pharmaceuticals containing an *N*-arylated secondary amine.

Figure 2 shows the yield of catalyst-free *N*-arylation of morpholine using various different polyfluorinated benzene derivatives.

Figure 3 shows the yield of catalyst-free *N*-arylation of various amines. General reaction conditions used in is: Amine (1.0 eq.), LiHMDS (1.0M in THF, 1.5 eq.) and benzene derivative (1.5 eq.) were mixed and heated. Compounds **3r**, **3s**, **3t**, **3ad**, **3ae**, **3af**, and **3ag** were synthesized by slightly modified procedures.

## Detailed description

The invention is defined by the appended claims. Any embodiment not falling under the scope of the appended claims does not form part of the invention.

## Definitions

The term "alkane" refers to saturated linear or branched carbohydrides of the general formula $C_nH_{2n+2}$.
The term "alkenyl" as used herein refers to a substituent derived from an alkene by removal of one -H. An alkene may be any acyclic carbonhydride comprising at least one double bond. Frequently, alkenyl will have the general formula $-C_nH_{2n-1}$.
The term "alkyl" refers to a substituent derived from an alkane by removal of one -H.
The term "alkynyl" as used herein refers to a substituent derived from an alkyne by removal of one -H. An alkyne may be any acyclic carbonhydride comprising at least one triple bond. Frequently, alkynyl will have the general formula $-C_nH_{2n-3}$.
The term "amino" as used herein refers to a substituent of the general formula

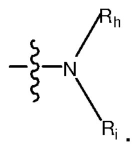

The waved line indicates the point of attachment of the substituent. Amino may thus for example be $-NH_2$ or -NH-.
The term "arene" as used herein refers to aromatic mono- or polycyclic carbonhydrides.
The term "aromatic" refers to a chemical substituent characterised by the following:

- contains a delocalized conjugated π system, most commonly an arrangement of alternating single and double bonds
- has a coplanar structure, with all the contributing atoms in the same plan
- the contributing atoms are arranged in one or more rings
- it contains a number of π delocalized electrons that is even, but not a multiple of 4.

The term "aromatic carbon atom" as used herein refers to a carbon atom, which contributes to an aromatic moiety. Consequently a "non-aromatic carbon atom" is a carbon atom which is not an integral part of an aromatic moiety. Accordingly, a non-aromatic carbon atom may optionally be linked to an aromatic moiety by a covalent bond. By way of examples, all carbon atoms of a phenyl group are considered "aromatic carbon atoms", however the carbon atoms of an alkyl group covalently linked to phenyl are considered "non-aromatic carbon atoms".
The term "aryl" as used herein refers to a substituent derived from an arene by removal of one -H from a C in the ring. Examples of useful aryls to be used with the present disclosure comprise phenyl, napthyl, anthracenyl, phenanthrenyl, and pyrenyl.
The term halogen as used herein refers to a substituent selected from the group consisting of -F, -Cl, -Br and -I.
The term "heteroalkenyl" refers to an alkenyl group, of which one or more carbon has been replaced by a heteroatom selected from S, O and N.
The term "heteroalkyl" refers to a straight- or branched-chain alkyl group, of which one or more carbon has been replaced by a heteroatom selected from S, O and N. Exemplary heteroalkyls include alkyl ethers, secondary and

tertiary alkyl amines, and alkyl sulfides.

The term "heteroaryl" as used herein refers to a substituent derived from an heteroarene by removal of one -H from an atom in the ring structure of said heteroarene. Heteroarenes are mono- or polycyclic aromatic compounds comprising one or more heteroatoms in the ring structure. Said heteroatoms are preferably selected from the group consisting of S, N and O. Non limiting examples of useful heteroaryls to be used with the present disclosure comprise azolyl, pyridinyl, pyrimidinyl, furanyl, and thiophenyl.

The term "non-aromatic heterocycle" refers to a mono- or polycyclic compound, which is not aromatic, and which comprises one or more heteroatom in the ring structure. Said heteroatoms are preferably selected from the group consisting of S, N and O. Examples of non-aromatic heterocycle includes but are not limited to pyrrolidine, piperidine, piperazine, morpholine, and thiomorpholine.

The term "phosphinyl" as used herein refers to a substituent of the general structure

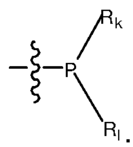

The waved line indicates the point of attachment of the substituent. Thus, phosphinyl may be $-PH_3$.

The term pKa as used herein refers to the negative logarithmic of the dissociation constant $K_a$ for an acid in a given solvent: $pK_a = -Log_{10} K_a$.

$K_a$, also called the *acidity constant*, is defined as:

$$K_a = \frac{[A^-][SH^+]}{[HA][S]}$$

for the reaction:

$$HA + S \rightleftharpoons A^- + SH^+$$

wherein S is the solvent and HA is an acid that dissociates into A-, known as the conjugate base of the acid, and a hydrogen ion which combines with a solvent molecule. When the concentration of solvent molecules can be taken to be constant, $K_a$, is:

$$K_a = \frac{[A^-][H^+]}{[HA]}$$

The term "substituted" as used herein in relation to chemical compounds refers to hydrogen group(s) being substituted with another moiety. Thus, "substituted with X" as used herein in relation to chemical compounds refers to hydrogen group(s) being substituted with X. Similarly, "substituted X" refers to X, wherein one hydrogen group has been substituted with another moiety. By way of example "substituted alkyl" refers to alkyl-R, wherein R is any moiety but -H.

The term "substituent" as used herein in relation to chemical compounds refers to an atom or group of atoms substituted in place of a hydrogen atom.

The term "strongly electron withdrawing substituents" as used herein refers to substituents with a Hammet meta substituent constant above 0.5, as described in Hanhsch 1991, and/or substituents having a double bond to oxygen of the linking atom.

The term "thioalkyl" as used herein refers to a substituent of the general formula -S-alkyl.

The term "thioaryl" as used herein refers to a substituent of the general formula -S-aryl.

The term "transition metal catalyst" refers to a compound capable of catalysing a chemical reaction, wherein said compound comprises a transition element or an ion of a transition element. A transition element is an element whose atom has an incomplete d sub-shell, or which can give rise to cations with an incomplete d sub-shell.

## Method for preparing an arylated amine

The present disclosure provides methods for preparing arylated amines. In particular, the methods of the present disclosure can be performed even in the absence of a transition metal catalyst.

The methods of the disclosure may comprise the steps of

a. Providing a nucleophile comprising an -NH- or and $-NH_2$ group, wherein the nucleophile for example may be any of the nucleophiles described herein below in the section "Nucleophile";

b. Providing an electrophile comprising an aryl substituted with at least one halogen and optionally further substituents, wherein the electrophile for example may be any of the electrophiles described herein below in the section "Electrophile",

c. Providing a base, which for example may be any of the bases described herein below in the section "Base"

d. Providing an organic solvent, wherein the solvent for example may be the solvent described herein below in the section "Solvent";

e. reacting said nucleophile with said electrophile in said organic solvent in the presence of the base, there by obtaining an arylated amine consisting of said aryl, wherein the halogen is substituted by said amine,

f. Optionally purifying the arylated amine.

The steps a., b., c. and d. may be performed in any suitable order. In one embodiment of the present disclosure, step e) comprises the sub-steps of

i) Reacting said nucleophile with said base,

ii) Reacting the product of sub-step i) with said electrophile,

wherein sub-steps i) and ii) are performed in the indicated order. This may in particular be the case in embodiments of the disclosure, where a strong base is used, e.g. a base, wherein the corresponding acid has a pKa above 45, such as above 49.

In case the nucleophile provided in a) comprises more than one -NH- and/or $-NH_2$ group, the amino group mentioned in a) is the amine acting as nucleophile in reaction to obtain the arylated amine.

As mentioned above one advantage of the methods according to the present disclosure is that the methods can be performed in the absence of a transition metal catalyst. Thus, it is preferred that step e. is performed in the absence of a transition metal catalyst. In some embodiments of the present disclosure, it may be preferred that the methods are performed in the absence of any transition metals, and in particular that step e. is performed in the absence of any transition metals. In particular, it may be preferred that the methods are performed in the absence of cupper, palladium and nickel, and in particular that step e. is performed in the absence of any cupper, palladium and nickel. Thus, the reaction, and in particular step e. is preferably performed in the absence of cupper, palladium and nickel in any oxidation state and any form.

Reacting said nucleophile with said electrophile may be done at any useful temperature. Thus, step e. may be performed at any useful temperature. One advantage of the methods of the disclosure is that the methods generally can be performed at temperatures, which are easy to handle, even in large scare. Thus, reacting said nucleophile with said electrophile may be performed at a temperature of at the most 120°C, such as at the most 110°C. Frequently even lower temperatures can be applied.

Reacting said nucleophile with said electrophile may be done for a time sufficient to allow the reaction. Thus, step e. may be performed for sufficient time to allow the reaction. One advantage of the methods of the disclosure is that generally a relative short time is required for the reactions. Thus, said nucleophile may typically be allowed to react with said electrophile for at the most one week. Frequently, the reaction may be even faster, thus in some embodiments of the disclosure, said nucleophile may be allowed to react with said electrophile for at the most 900 min, such as for at the most 720 min, such as for the most 180 min, for example for in the range of 5 to 900 min or in the range of 5 to 720 min.

## Nucleophile

The methods of the disclosure involve reacting a nucleophile and an electrophile. The nucleophile useful with the present disclosure must comprise an -NH- or and $-NH_2$ group. In general, said nucleophile comprises an -NH- or an $-NH_2$ group directly linked to only non-aromatic carbon atoms.

Thus, in one embodiment of the present disclosure, the nucleophile contains an -NH-group, which is covalently linked to two non-aromatic carbon atoms. Said non-aromatic carbon atoms may for example be a carbon atom of an alkyl or of an alkyl substituted with one or more substituents. It follows that the nucleophile thus may be a secondary amine.

In another embodiment of the present disclosure, said nucleophile comprises an $-NH_2$ group, which is covalently

linked to a non-aromatic carbon atom. For example said nucleophile may be alkyl-$NH_2$ or alkyl-$NH_2$, wherein said alkyl is substituted with one or more substituents. It follows that the nucleophile may be a primary amine.

In one embodiment of the present disclosure, the nucleophile may be a compound of the formula I:

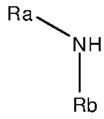 (I),

wherein

$R_a$ and $R_b$ individually are selected from the group consisting of -H and alkyl, wherein said alkyl optionally may be substituted with aryl or substituted aryl with the proviso that only one of $R_a$ and $R_b$ may be -H; or

$R_a$ and $R_b$ together forms a non-aromatic heterocycle, which optionally may comprise one or more heteroatoms, wherein said heterocycle optionally may be substituted.

Thus, in one embodiment of the present disclosure, $R_a$ may be selected from the group consisting of -H, $C_{1-50}$-alkyl, $C_{1-10}$-alkyl-aryl. For example, $R_a$ may be selected from the group consisting of -H, $C_{1-10}$-alkyl and $C_{1-10}$-alkyl-phenyl. In particular, $R_a$ may be selected from the group consisting of -H, $C_{1-5}$-alkyl and-$CH_2$-phenyl.

In one embodiment of the present disclosure, $R_b$ may be selected from the group consisting of $C_{1-50}$-alkyl, $C_{1-10}$-walkyl-aryl. For example, $R_b$ may be selected from the group consisting of $C_{1-10}$-alkyl and $C_{1-10}$-walkyl-phenyl. In particular, $R_b$ may be selected from the group consisting of $C_{1-5}$-alkyl and-$CH_2$-phenyl.

The nucleophile may also be a compound of formula I, wherein $R_a$ and $R_b$ together forms a non-aromatic heterocycle, which optionally may comprise one or more heteroatoms, and which may be substituted.

Thus, it is comprised in the disclosure that the nucleophile may be 4 to 10 membered non-aromatic heterocycle comprising at least one N atom. For example, the nucleophile may be a 4 to 10 membered non-aromatic heterocycle comprising 1 or 2 heteroatoms, wherein at least one is an N atom. Said 4 to 10 membered non-aromatic heterocycle may for example be a monocyclic or a bicyclic non-aromatic heterocycle.

Said 4 to 10 membered non-aromatic heterocycle comprising at least one N atom may optionally be substituted with one or more substituents, e.g. with one or more substituents selected from the group consisting of aryl, substituted aryl, heteroaryl, substituted heteroaryl, alkyl, substituted alkyl, alkenyl, substituted alkenyl, alkynyl or substituted alkynyl. For example, said 4 to 10 membered non-aromatic heterocycle may be substituted with one or more substituents selected from the group consisting of $C_{1-6}$-alkyl and aryl. For example said 4 to 10 membered non-aromatic heterocycle may be substituted with one substituent selected from the group consisting of phenyl and $C_{1-3}$-alkyl, e.g. methyl.

It is also comprised within the disclosure that the nucleophile may be a 4 to 8 membered non-aromatic heterocycle comprising at least one N atom. Thus, the nucleophile may be a 4 to 8 membered non-aromatic heterocycle comprising only 1 heteroatom, wherein said heteroatom is an N atom. The term "comprising only 1 heteroatom" as used herein refers to that the ring of said heterocycle consists of carbon atoms and said one heteroatom. Said carbon atoms may optionally be substituted. Thus, said 4 to 8 membered non-aromatic heterocycle comprising at least one N atom may optionally be substituted with one or more substituents, e.g. with one or more substituents selected from the group consisting of aryl, substituted aryl, heteroaryl, substistuted heteroaryl, alkyl, substituted alkyl, alkenyl, substituted alkenyl, alkynyl or substituted alkynyl. For example, said 4 to 8 membered non-aromatic heterocycle may be substituted with one or more substituents selected from the group consisting of $C_{1-6}$-alkyl and aryl. For example said 4 to 8 membered non-aromatic heterocycle may be substituted with one substituent selected from the group consisting of phenyl and $C_{1-3}$-alkyl, e.g. methyl. Said non-aromatic heterocycle may for example be selected from the group consisting of piperazine, morpholine, tetrahydroisoquinoline, dioxa-azaspiro-decane, piperidine, and thiomorpholine.

In one embodiment of the disclosure the nucleophile is a compound of the formula II

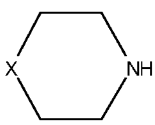 (II)

wherein X is $NR_c$, NH, O or S.

$R_c$ may for example be aryl, substituted aryl, heteroaryl, substistuted heteroaryl, alkyl, substituted alkyl, alkenyl, substituted alkenyl, alkynyl, substituted alkynyl, 3 to 8 membered cycloalkyl or 3-8 membered nonaromatic heterocycle.

For example, $R_c$ may be selected from the group consisting of aryl, $C_{1-10}$-alkyl, $C_{2-10}$-alkenyl, $C_{2-10}$-alkynyl and 3-8 membered nonaromatic heterocycle. For example, $R_c$ may be selected from the group consisting of phenyl, and $C_{1-10}$-alkyl.

In one embodiment of the present disclosure, the nucleophile is selected from the group consisting of: pyrrolidine, N-methylpiperazine, 1-methylpiperazine, 1,4-dioxa-8-azaspiro[4.5]decane, piperidine, piperazine, morpholine, thiomorpholine, 1-phenylpiperazine, N-ethylbutan-1-amine, 1,2,3,4-tetrahydroisoquinoline, dibenzylamine, N-methyl-benzylamine and benzylamine.

In one preferred embodiment of the present disclosure, the nucleophile is selected from the group consisting of N-methylpiperazine, piperazine, morpholine and 1,2,3,4-tetrahydroisoquinoline. In one preferred embodiment of the present disclosure, the nucleophile is selected from the group consisting of N-methylpiperazine, piperazine and morpholine.

The nucleophile may also be salts of any of the aforementioned nucleophiles. For example, the nucleophile may be a salt of any of the aforementioned nucleophiles with various inorganic or organic acids. In embodiments of the disclosure, where the arylated amine is for pharmaceutical use, said salt may be a pharmaceutically acceptable salt.

**Electrophile**

The methods of the disclosure involve reacting a nucleophile and an electrophile. The electrophile useful with the present disclosure comprises or consists of an aryl substituted with at least one halogen and optionally further substituents. Preferably, the electrophile is aryl substituted with at least two substituents, wherein the first substituent is halogen and the second substituent is selected from the group of substituents consisting of halogen, aryl, substituted aryl, alkenyl, substituted alkenyl, heteroalkenyl, alkyl, substituted alkyl, heteroalkyl, alkoxy, substituted alkoxy, amino, thioalkyl, substituted thioalkyl, thioaryl, substituted thioaryl, heteroaryl, and phosphinyl, with the proviso that said aryl is substituted with at the most 4 halogens. In addition to said first and second substituents, said aryl may be substituted with one or more additional substituents, which preferably are selected from the same group of substituents as the second substituents.

In one embodiment of the present disclosure, the second substituent may also be selected from the group of substituents consisting of halogen, aryl, substituted aryl, alkyl, substituted alkyl, alkoxy, substituted alkoxy, amino, thioalkyl, substituted thioalkyl, thioaryl, substituted thioaryl, heteroaryl, and phosphinyl, with the proviso that said aryl is substituted with at the most 4 halogens.

The aryl may be any aryl, for example the aryl may be selected from the group consisting phenyl and naphtalenyl. In preferred embodiments of the disclosure the aryl is phenyl. Thus, the electrophile may be aryl (e.g. phenyl) substituted with halogen (e.g. -F) and a $2^{nd}$ substituent. The electrophile may be aryl (e.g. phenyl) substituted with halogen (e.g. -F) and a $2^{nd}$ and a $3^{rd}$ substituent. The electrophile may be aryl (e.g. phenyl) substituted with halogen (e.g. -F) and a $2^{nd}$, a $3^{rd}$ and a $4^{th}$ substituent. The electrophile may be aryl (e.g. phenyl) substituted with halogen (e.g. -F) and a $2^{nd}$, a $3^{rd}$, a $4^{th}$ and a $5^{th}$ substituent. The electrophile may be aryl (e.g. phenyl) substituted with halogen (e.g. -F) and a $2^{nd}$, a $3^{rd}$, a $4^{th}$, a $5^{th}$, and a $6^{th}$ substituent. Said $2^{nd}$, $3^{rd}$, $4^{th}$, $5^{th}$ and $6^{th}$ substituent may for example be any of the substituents described herein below in this section.

In one embodiment of the present disclosure, the electrophile may be a chloro- or fluorobenzene derivative without strongly electron withdrawing substituents.

The electrophile may also be a weakly or non-electron deficient chloro- or fluorobenzene derivative.

The electrophile may also be phenyl substituted with only 2 halogens and optionally a $3^{rd}$, $4^{th}$, $5^{th}$ and/or $6^{th}$ substituent. Thus, in such embodiment of the present disclosure, the phenyl is covalently linked to exactly two halogens. Said halogen may in particular be -F. In addition, said phenyl may be substituted with a $3^{rd}$, $4^{th}$, $5^{th}$ and/or $6^{th}$ substituent, which is not halogen. The $3^{rd}$, $4^{th}$, $5^{th}$ and/or $6^{th}$ substituent may be any of the substituents described herein below in this section.

The electrophile may also be phenyl substituted with only 1 -F and optionally a $2^{nd}$, $3^{rd}$, $4^{th}$, $5^{th}$ and/or $6^{th}$ substituent. Thus, in such embodiment of the present disclosure, the phenyl is covalently linked to exactly one -F. In addition, said phenyl may be substituted with a $2^{nd}$, $3^{rd}$, $4^{th}$, $5^{th}$ and/or $6^{th}$ substituent, which is not -F, and preferably not halogen. The $2^{nd}$, $3^{rd}$, $4^{th}$, $5^{th}$ and/or $6^{th}$ substituent may be any of the substituents described herein below in this section.

Said $2^{nd}$, $3^{rd}$, $4^{th}$, $5^{th}$ and $6^{th}$ substituent may be individually selected from the group of consisting of aryl,

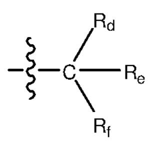

,

$R_gO$-,

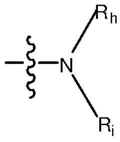,

Azolyl- Br-, Cl-, F-, $F_3C$, $R_jS$-, and

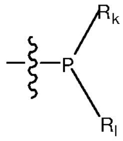,

wherein $R_d$, $R_e$, $R_f$, $R_g$, $R_h$, $R_i$, $R_j$, $R_k$ and $R_l$ individually are selected from the group consisting of -H, aryl, substituted aryl, heteroaryl, substituted heteroaryl, alkyl, substituted alkyl, alkenyl, substituted alkenyl, alkynyl and substituted alkynyl. The waved line indicates the point of attachment of the substituent.

Said 2$^{nd}$, 3$^{rd}$, 4$^{th}$, 5$^{th}$ and 6$^{th}$ substituent may be individually selected from the group consisting of

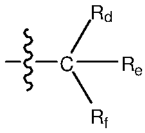,

$R_gO$-,

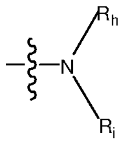,

Azolyl-, Cl-, F-, and $R_jS$-, wherein $R_d$, $R_e$, $R_f$, $R_g$, $R_h$, $R_i$ and $R_j$ individually are selected from the group consisting of -H, aryl, substituted aryl, heteroaryl, substituted heteroaryl, alkyl, substituted alkyl, alkenyl, substituted alkenyl, alkynyl and substituted alkynyl. The waved line indicates the point of attachment of the substituent.

Said 2$^{nd}$, 3$^{rd}$, 4$^{th}$, 5$^{th}$ and 6$^{th}$ substituent may be individually selected from the group consisting of $R_gO$-,

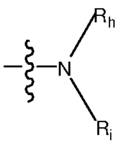,

Cl-, F-, and $R_jS$-,
wherein $R_g$, $R_h$, $R_i$ and $R_j$ individually are selected from the group consisting of -H, aryl, substituted aryl, heteroaryl, substituted heteroaryl, alkyl, substituted alkyl, alkenyl, substituted alkenyl, alkynyl and substituted alkynyl. The waved line indicates the point of attachment of the substiuent.

Said 2$^{nd}$, 3$^{rd}$, 4$^{th}$, 5$^{th}$ and 6$^{th}$ substituent may be individually selected selected from the group consisting of Cl-, F-, and $R_jS$-,
wherein $R_j$ is selected from the group consisting of -H, aryl, substituted aryl, heteroaryl, substituted heteroaryl, alkyl, substituted alkyl, alkenyl, substituted alkenyl, alkynyl and substituted alkynyl.

In one embodiment of the present disclosure, electrophile is aryl (e.g. phenyl) substituted with 2 to 4 substituents, wherein all of said substituents are halogen.

The first substituent may be halogen. Preferably, said halogen is selected from the group consisting of -F, -Cl and -Br, more said first substituent is selected from the group consisting of -F and -Cl, for example the first substituent is -F.

In embodiments of the disclosure, wherein one or more of the 2nd, 3rd, 4th, 5th and 6th substituents are halogen, then said halogen may be any halogen, preferably said halogen is selected from the group consisting of -F, -Cl and -Br, more preferably said halogen is selected from the group consisting of -F and -Cl.

In one embodiment of the disclosure the electrophile is aryl (e.g. phenyl) substituted with one or more substituents, where all of said substituents are selected from the group consisting of -F and -Cl.

In embodiments of the disclosure, wherein one or more of $R_d$, $R_e$, $R_f$, $R_g$, $R_h$, $R_i$, $R_j$, $R_k$ and $R_l$ is aryl, said aryl may be selected from the group consisting of phenyl, napthyl, indenyl, and fluorenyl, in particular said aryl may be phenyl.

In embodiments of the disclosure, wherein one or more of $R_d$, $R_e$, $R_f$, $R_g$, $R_h$, $R_i$, $R_j$, $R_k$ and $R_l$ is substituted aryl, said substituted aryl may be selected from the group consisting of phenyl, napthyl, indenyl, and fluorenyl substituted with one or more selected from the group consisting of -OH, aryl, $C_{1-6}$-alkyl, $C_{2-6}$-alkenyl and $C_{2-6}$-alkynyl, in particular said substituted aryl may be phenyl substituted with one or more selected from the group consisting of phenyl, -OH, $C_{1-6}$-alkyl, $C_{2-6}$-alkenyl and $C_{2-6}$-alkynyl.

In embodiments of the disclosure, wherein one or more of $R_d$, $R_e$, $R_f$, $R_g$, $R_h$, $R_i$, $R_j$, $R_k$ and $R_l$ is heteroaryl, said heteroaryl may individually for example be selected from the group consisting of tetrazolyl, imidazolyl, anthracenyl, phenanthrenyl, fluorenyl, pentalenyl, azulenyl, biphenylenyl, furanyl, triazolyl, pyranyl, thiadiazinyl, benzothiophenyl, dihydro-benzo[b]thiophenyl, xanthenyl, isoindanyl, benzhydryl, acridinyl, benzisoxazolyl, quinolinyl, isoquinolinyl, phteridinyl, azepinyl, diazepinyl, imidazolyl, thiazolyl, quinolyl, carbazolyl, pyridinyl, pyridazinyl, imidazolyl, pyrimidinyl, pyrazolyl, triazolyl, pyrazinyl, quinolyl, isoquinolyl, tetrazolyl, furyl, thienyl, isoxazolyl, thiazolyl, oxazolyl, isothiazolyl, pyrrolyl, indolyl, benzimidazolyl, benzofuranyl, cinnolinyl, indazolyl, indolizinyl, phthalazinyl, triazinyl, isoindolyl, purinyl, oxadiazolyl, thiadiazolyl, furazanyl, benzofurazanyl, benzothiophenyl, benzotriazolyl, benzothiazolyl, benzoxazolyl, quinazolinyl, quinoxalinyl, naphthyridinyl, dihydroquinolyl, tetrahydroquinolyl, dihydroisoquinolyl, tetrahydroisoquinolyl, benzofuryl, furopyridinyl, pyrolopyrimidinyl, azaindolyl, imidazolyl, pyrazolyl, pyridyl, pyridazinyl, pyrazinyl, pyrimidinyl, tetrazolyl, pyrazolinyl, and pyrazolidinyl.

In embodiments of the disclosure, wherein one or more of $R_d$, $R_e$, $R_f$, $R_g$, $R_h$, $R_i$, $R_j$, $R_k$ and $R_l$ is substituted heteroaryl, said substituted heteroaryl may individually for example be any of the aforementioned heteroaryl substituted with one or more selected from the group consisting of -OH, aryl, $C_{1-6}$-alkyl, $C_{2-6}$-alkenyl and $C_{2-6}$-alkynyl, in particular substituted with one or more selected from the group consisting of phenyl,-OH, $C_{1-6}$-alkyl, $C_{2-6}$-alkenyl and $C_{2-6}$-alkynyl.

In embodiments of the disclosure, wherein one or more of $R_d$, $R_e$, $R_f$, $R_g$, $R_h$, $R_i$, $R_j$, $R_k$ and $R_l$ is alkyl, said alkyl may individually for example be $C_{1-10}$-alkyl, such as $C_{1-6}$-alkyl, for example $C_{1-3}$-alkyl.

In embodiments of the disclosure, wherein one or more of $R_d$, $R_e$, $R_f$, $R_g$, $R_h$, $R_i$, $R_j$, $R_k$ and $R_l$ is substituted alkyl, said alkyl may individually for example be $C_{1-10}$-alkyl, $C_{1-6}$-alkyl or $C_{1-3}$-alkyl.

In embodiments of the disclosure, wherein $R_d$, $R_e$, $R_f$, $R_g$, $R_h$, $R_i$, $R_j$, $R_k$ and/or $R_l$ is alkyl, said alkyl may for example be $C_{1-10}$-alkyl, such as $C_{1-6}$-alkyl, for example $C_{1-3}$-alkyl, substituted with one or more selected from the group consisting of -OH and aryl, in particular substituted with one or more selected from the group consisting of phenyl and -OH.

In embodiments of the disclosure, wherein one or more of $R_d$, $R_e$, $R_f$, $R_g$, $R_h$, $R_i$, $R_j$, $R_k$ and $R_l$ is substituted alkenyl, said alkenyl may individually for example be $C_{2-10}$-alkenyl, $C_{2-6}$-alkenyl or $C_{2-3}$-alkenyl.

In embodiments of the disclosure, wherein $R_d$, $R_e$, $R_f$, $R_g$, $R_h$, $R_i$, $R_j$, $R_k$ and/or $R_l$ is alkenyl, said alkenyl may for example be $C_{2-10}$-alkenyl, such as $C_{2-6}$-alkenyl, for example $C_{2-3}$-alkenyl, substituted with one or more selected from the group consisting of -OH and aryl, in particular substituted with one or more selected from the group consisting of phenyl and -OH.

In embodiments of the disclosure, wherein one or more of $R_d$, $R_e$, $R_f$, $R_g$, $R_h$, $R_i$, $R_j$, $R_k$ and $R_l$ is substituted alkynyl, said alkynyl may individually for example be $C_{2-10}$-alkynyl, $C_{2-6}$-alkynyl or $C_{2-3}$-alkynyl.

In embodiments of the disclosure, wherein $R_d$, $R_e$, $R_f$, $R_g$, $R_h$, $R_i$, $R_j$, $R_k$ and/or $R_l$ is alkynyl, said alkynyl may for example be $C_{2-10}$-alkynyl, such as $C_{2-6}$-alkynyl, for example $C_{2-3}$-alkynyl, substituted with one or more selected from the group consisting of -OH and aryl, in particular substituted with one or more selected from the group consisting of phenyl and -OH.

In one embodiment of the present disclosure, said 2nd, 3rd, 4th, 5th and 6th substituent may be individually selected from the group of consisting of -NH-alkenyl, -S-alkenyl,-O-alkenyl, -NH-(CH)n-NH, $-O-(CH)_n-NH$, -S-(CH)n-NH, -NH-N-(CH)n-CH2, -NH-N=NH,$-NH-(CH_2)_n-CH_3$,$-(CH_2)_n-NH-(CH_2)_m-CH_3$, wherein n and m are individually 0 or an integer.

In particular, one substituent may be fused directly with the arene. In other embodiments of the present disclosure, the substituent is not fused with the arene.

Two of said 2nd, 3rd, 4th, 5th and 6th substituents may be fused and thereby forming a ring. Preferably, such fused substituents form a ring selected from the group consisting of pyrrole, furan, thiophene, pyrazole, oxazole, thiazole, imidazole, 1,2,3-triazole, 3,4-dihydro-pyrrole, and 2,3-dihydro-pyrrole rings. In one embodiment of the present disclosure, the electrophile is an indole substituted with at least one halogen on the arene. In another embodiment of the present disclosure, the electrophile is a benzofuran substituted with at least one halogen on the arene. In yet another embodiment of the present disclosure, the electrophile is a benzothiophene substituted with at least one halogen on the arene. In yet

another embodiment of the present disclosure, the electrophile is an indazole substituted with at least one halogen on the arene. In yet another embodiment of the present disclosure, the electrophile is a benzoxazole substituted with at least one halogen on the arene. In yet another embodiment of the present disclosure, the electrophile is a benzothiazole substituted with at least one halogen on the arene. In yet another embodiment of the present disclosure, the electrophile is a benzimidazole substituted with at least one halogen on the arene. In yet another embodiment of the present disclosure, the electrophile is a benzotriazole substituted with at least one halogen on the arene. In yet another embodiment of the present disclosure, the electrophile is an isoindoline substituted with at least one halogen on the arene. In yet another embodiment of the present disclosure, the electrophile is an indoline substituted with at least one halogen on the arene.

In embodiments of the disclosure, the electrophile is selected from the group consisting of indole, benzothiophene, benzofuran, benzimidazole, benzoxazole, benzothiazole, indazole, benzotriazole, isoindoline and indoline (shown below), substituted with at least one halogen on the benzene moiety and optionally further substituents. In the cases where a secondary amine is present in the ring fused with the aryl, said secondary amine is preferably first transformed into a tertiary amine, such as being alkylated, alkenylated or arylated.

The electrophile may for example be selected from the group consisting of the following compounds, wherein said compounds are substituted with at least one halogen on the benzene moiety and optionally further substituents:

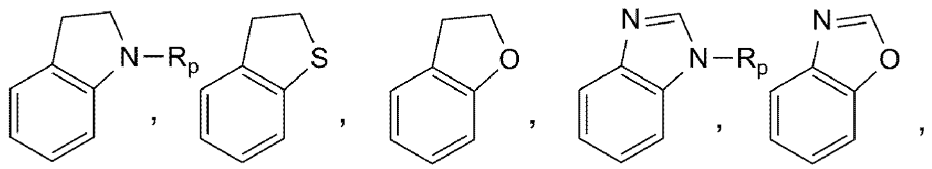
, , , , ,

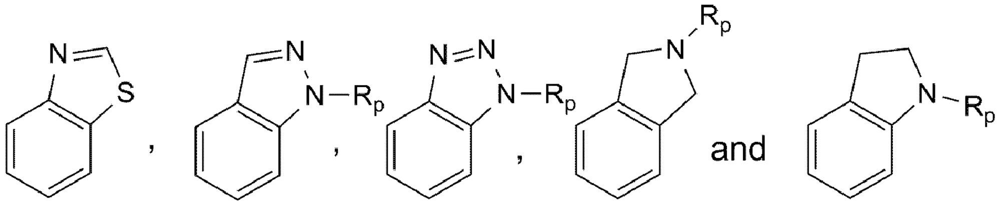
, , , and

wherein $R_p$ is selected from the group consisting of hydrogen, alkyl, alkenyl, carbamate, sulfone, benzyl, acetyl, benzoyl, carbobenzyloxy, p-methoxybenzyl carbonyl, tert-butyloxycarbonyl, p-methoxybenzyl, 3,4-dimethoxybenzyl, p-methoxyphenyl, tosyl, and trichloroethyl chloroformate. In some embodiments of the present disclosure, $R_p$ is not hydrogen.

In other embodiments of the present disclosure, none of the said 2$^{nd}$, 3$^{rd}$, 4$^{th}$, 5$^{th}$ and 6$^{th}$ substituents are fused with each other.

In one embodiment of the present disclosure, the electrophile is phenyl substituted with first substituent selected from the group consisting of -F and -Cl, and with a 2$^{nd}$ substituent selected from the group consisting of -F, -Cl, $C_{1-3}$-alkoxy, $C_{1-3}$-alkyl, thioaryl and phenyl, wherein said thioaryl may be substituted with up to 3 $C_{1-3}$-alkyl.

In one embodiment of the present disclosure, the electrophile is phenyl substituted with first substituent selected from the group consisting of -F, -Cl, $C_{1-3}$-alkoxy, $C_{1-3}$-alkyl, thioaryl and phenyl, wherein said thioaryl may be substituted with up to 3 $C_{1-3}$-alkyl.

In one embodiment of the present disclosure, the electrophile is selected from the group consisting of tetrafluorobenzene, trifluorobenzene, difluorobenzene, fluoro-chlorobenzene, dichloro-fluorobenzene, trichlorobenzene, dichlorobenzene, chloro-difluorobenzene, methyl-difluorobenzene, methyl-chloro-fluorobenzene, methoxy-fluorobenzene, di-methyl-thiophenol-fluorobenzene, fluoro-1,1'-biphenyl, N-benzyl-3,5-difluoro-N-methylaniline and bi-phenyl.

In one embodiment of the present disclosure, the electrophile is selected from 2,3-dichloro-fluorobenzene, 1,2,3-trichlorobenzene, 2-(4,2-di-methyl-thiophenol-yl)-fluorobenzene, and 2-(4,2-di-methyl-thiophenol-yl)-chlorobenzene.

The electrophile may be a product of an organic synthesis and may thus be considered an intermediate. For example, the electrophile may be a product of a cross coupling reaction.

**Base**

The methods of the present disclosure involve reacting a nucleophile and an electrophile in the presence of a base. Preferably said base is a base, wherein the corresponding acid has a pKa above 29, such as at least 30 in DMSO. It may also be preferred that said base has a pKa above 25, preferably at least 26 in THF. Thus, it is generally preferred that the base is not a weak base, for example the base is preferably not $Cs_2CO_3$.

In some embodiments of the disclosure the base may be a base, wherein the corresponding acid has a pKa above 32 in DMSO. In some embodiments of the present disclosure, the base may be a base, wherein the corresponding acid has a pKa above 26 in THF.

In some embodiments of the disclosure it may be preferred that the base is not too strong. Thus, in some embodiments of the present disclosure, it is preferred that the base is a base, wherein the corresponding acid has a pKa in the range of 29 to 49, such as in the range of 29 to 45. In other embodiments of the present disclosure, the base is a base, wherein the corresponding acid has a pKa in the range of 32 to 49, such as in the range of 32 to 45. Aforementioned pKa is preferably determined in DMSO.

pKa may be determined by any conventional method. pKa values in DMSO is preferably measured up to the value of 35, and values above 35 may be extrapolated as described in Bordwell, Acc. Chem. Res. 1988, 21, 456-463.

The corresponding acid to butyllithium (BuLi) has a pKa 50, and may thus in some embodiments of the present disclosure be less preferable. Thus, in some embodiments of the present disclosure, the base is a base, wherein the corresponding acid has a pKa above 29, for example above 32, with the proviso that the base is not BuLi.

In some embodiments of the present disclosure, the base is a metal hydride, such as an alkali metal hydride. In some embodiments of the present disclosure, the base is selected from the group consisting of lithium hydride, sodium hydride, potassium hydride, cesium hydride, magnesium hydride, calcium hydride, lithium aluminium hydride, sodium aluminium hydride, potassium aluminium hydride, lithium borohydride, sodium borohydride and potassium borohydride.

In one embodiment of the present disclosure, the base is selected from the group consisting of lithium bis(trimethylsilyl)amide (LiHMDS), sodium bis(trimethylsilyl)amide (NaHMDS), potassium bis(trimethylsilyl)amide (KHMDS), lithium 2,2,6,6,-tertmethylpiperidide (LiTMP), and BuLi.

In some embodiments of the present disclosure, the base is a non-nucleophilic base, i.e. a base only acting as a nucleophile in the removal of protons. Typical non-nucleophilic bases are sterically hindered and bulky, preventing them from attacking as nucleophiles. Hence, protons can attach to the basic center of the base but alkylation and complexation is inhibited. Examples of non-nucleophilic bases are lithium diisopropylamide (LDA), LiTMP and silicon-based amides such as LiHMDS, NaHMDS and KHMDS. Generally, non-nucleophilic bases are sterically hindered and bulky, and thus the base may be a base having a $M_w$ of at least 120 g/mol, preferably of at least 130 g/mol, such as of at least 140 g/mol. More preferably the base has aforementioned Mw and aforementioned pKa. Thus, it may be preferred that the base has:

- a pKa above 29 in DMSO and/or a pKa in THF above 25; and
  a Mw of at least 120 g/mol or a $M_w$ of at least 140 g/mol.

In other embodiments of the present disclosure, the base is selected from the group consisting of LiHMDS, NaHMDS, KHMDS and LiTMP.

**Solvent**

The methods of the present disclosure involve reacting a nucleophile and an electrophile in a solvent and in the presence of a base. The solvent may be any organic solvent.

In one embodiment of the present disclosure, the solvent may be chosen according to the base used in the particular reaction. Thus, the solvent may be an organic solvent, which is stable in the presence the base employed under the reaction conditions employed.

In addition, it is preferred that the solvent is a liquid at the reaction temperature.

It is preferred that the solvent is a solvent that only contain protons with a pKa above 35.1 in DMSO. In one embodiment of the present disclosure, the solvent is a solvent that only contains protons with a pKa above 32 in DMSO. In one embodiment of the present disclosure, the solvent is not DMSO. Thus, the solvent may be a solvent that only contains protons with a pKa above 32 in DMSO, with the proviso that the solvent is not DMSO.

In one embodiment of the present disclosure, the solvent is a solvent that does not contain any carbonyl groups. In one embodiment of the present disclosure, the solvent is a solvent that does not contain any sulfoxide groups.

The solvent may for example be selected from the group consisting of ethers, alkanes, benzene and substituted benzene.

Ethers useful as solvent include any ether. In particular, the ether may be an ether, which only contain protons with a pKa above 32, for example above 35.1 in DMSO. It may further be preferred that the ether is a liquid at the reaction temperature. It may further be preferred that the ether does not contain any carbonyl groups. The ether may for example be selected from the group consisting of tetrahydrofuran (THF), dioxane, dimethoxyethane (DME), 2-methyl-tetrahydrofuran (2-Me-THF), and diethoxyethane.

Alkanes useful as solvent include any alkane. In particular, the alkane may be an alkane, which only contain protons with a pKa above 32, for example above 35.1 in DMSO. It may further be preferred that the alkane is a liquid at

the reaction temperature. It may further be preferred that the alkane does not contain any carbonyl groups. The alkane may be a linear, branched or cyclic alkane, e.g. a $C_{4-20}$ linear, branched or cyclic alkane. For example, the alkane may be methylcyclohexane.

Substituted benzenes useful as solvent include any substituted benzene. In particular, the substituted benzene may be a substituted benzene, which only contain protons with a pKa above 32, for example above 35.1 in DMSO. It may further be preferred that the substituted benzene is a liquid at the reaction temperature. It may further be preferred that the substituted benzene does not contain any carbonyl group. The substituted benzene is in general different from the electrophile used in the reaction. However in some embodiments of the present disclosure, the electrophile may also be used as solvent. For example the substituted benzene may be substituted with one or more substituents selected from the group consisting of $C_{1-3}$-alkyl. In addition or alternatively, the benzene may be substituted with up to 1 -Cl.. The substituted benzene may for example be selected from the group consisting of toluene, xylene and chlorobenzene.

**Arylated amine**

The arylated amine to be prepared by the methods according to the disclosure may be any of the electrophiles described herein above, wherein in place of the first substituent, the aryl of the electrophile is covalently N-linked to one of the nucleophiles described herein above.

In one embodiment of the disclosure the arylated amine is selected from the group of compounds shown in figure 2 as compounds 3c,3d, 3e, 3f, 3g, 3h, 3i, 3j, 3l and 3m.

In one embodiment of the disclosure the arylated amine is selected from the group of compounds shown in figure 3 as compounds 3n, 3o, 3p, 3q, 3r, 3s, 3t, 3u, 3v, 3w, 3x, 3y, 3z, 3aa, 3ab, 3ac, 3ad, 3ae, 3af and 3ag.

In one embodiment of the present disclosure, the arylated amine is selected from the group of compounds shown in figure 1.

The arylated amine may be used in further organic synthesis, and thus in some embodiments of the present disclosure, the arylated amine may be an intermediate. Thus, the arylated amine may be a substrate for further functionalization e.g. through cross coupling reactions.

In other embodiments of the present disclosure, the arylated amine may be a final product. The arylated amine may be purified by any conventional method including for example extraction, precipitation, crystallisation, distillation and/or chromatography.

Examples

Examples not falling under the scope of the appended claims do not form part of the invention.

**Example 1**

In the following examples, N-arylation of amines was performed as follows unless otherwise specified:

To a vial was added amine nucleophile (1.0 mmol) and base. If nothing else is specified the base employed was LiHMDS (1.0 M in THF, 1.5 mL). The vial was sealed and stirred at the designated temperature for 10 minutes. To this mixture was then added fluoro electrophile (1.5 mmol) at room temperature. The reaction was stirred at the designated temperature until judged complete by HPLC. The reaction was then quenched by addition of solid $NaHCO_3$ and loaded directly onto a silica gel column. The product was purified by flash chromatography on silica gel using a suitable mixture of ethyl acetate and heptane as eluent.

**Example 2**

N-arylation of compound 1a (N-methylpiperazine) with compound 2 (1,3,5-trifluorobenzene) was performed using the following general reaction conditions: **1a** (0.2 mmol) and the base (0.5 mmol) was mixed in solvent (0.5 mL) at room temperature. Different solvents, bases and temperatures were tested.

After 10 minutes 2 (0.6 mmol) was added and reaction heated to temperature and stirred for 12 h. Yields assessed by HPLC. The results are shown in table 1 below.

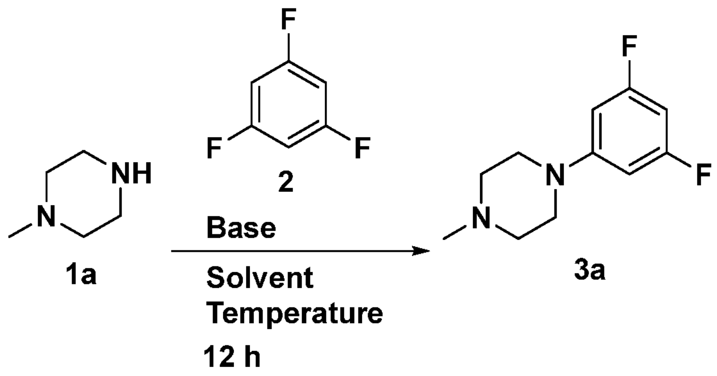


1a 2 3a

Table 1

| Entry | Base | $M_w$ (g/mol) | p$K_a$ DMSO | p$K_a$ THF | Solvent | Temperature | Yield **3a** (%)[a] |
|---|---|---|---|---|---|---|---|
| A | None | - | - | - | THF | 50°C | 0 |
| B | $Cs_2CO_3$ | 325.82 | ** | ** | THF | 50°C | 0 |
| C | $LiO^tBu$ | 80.05 | 29 | | THF | 50°C | <5** |
| D | LiHMDS | 167.33 | 30*** | 26 | THF | 50°C | >95 |
| E* | LiTMP | 147.19 | 37 | | THF | 90°C | 42 (**3b**) |
| F | LiHMDS | 167.33 | 30*** | 26 | DME | 50°C | 73 |
| G | LiHMDS | 167.33 | 30*** | 26 | 2-Me-THF | 50°C | 51 |

[a] Evaluated by HPLC.
*1-fluoro-3-methoxybenzene used instead of 1,3,5-trifluorobenzene.
** pKa of $Cs_2CO_3$ is expected to be lower than 29 in DMSO and 25 in THF.
*** pKa of LiHMDS has been reported to be 30 in DMSO (http://www.d-bernier.fr/pKa.php)

The reaction was successfully performed using LiOtBu as base, but it was significantly less efficient than using some of the other bases.

**Example 3**

The scope of arylation reactions with respect to the fluorobenzenes in reaction with morpholine **1b** was investigated, and the results are provided in Figure 2. Polyfluorinated benzene derivates perform well in these reactions as seen from the formation of products **3c-3m**, which was isolated in yields up to 93%. Important notions about these reactions are the ability to achieve mono-substitution in the presence of additional fluorine atoms as well as the high degree of regioselectivity. For example, product **3c** is formed as a single regioisomer even though the starting 1,2,3,5-tetrafluorobenzene contains no less than three distinct fluorine atoms. Difluorinated benzenes can also be employed as electrophiles in the reactions as illustrated by the formation of **3f** and **3g** isolated in 81% and 86% yield, respectively.

The general reaction conditions used in this example: Morpholine 1b (1.0 mmol), LiHMDS (1.0M in THF, 1.5 mL) and fluorobenzene derivative (1.5 mmol) was mixed and heated. Details regarding the individual reactions are given below.

The structure of the compounds is provided in figure 2.

Compound **3c**, 4-(2,3,5-trifluorophenyl)morpholine:

Synthesized from 1,2,3,5-tetrafluorobenzene according to the general procedure. After 3 hours at room temperature the compound **3c** was isolated in 70% yield as an off-white solid. $^1$H NMR ($CDCl_3$) δ ppm 6.58 - 6.48 (m, 1H), 6.41 (ddt, *J* = 11.2, 5.6, 2.6 Hz, 1H), 3.90 - 3.81 (m, 4H), 3.12 - 3.07 (m, 4H). $^{13}$C NMR ($CDCl_3$) δ ppm 157.9 (ddd, *J* = 243.5, 13.4, 3.3 Hz), 151.3 (ddd, *J* = 247.4, 15.3, 13.5 Hz), 142.1 (ddd, *J* = 10.5, 6.8, 3.5 Hz), 140.5 (ddd, *J* = 242.9, 14.1, 4.6 Hz), 100.7 (dt, *J* = 26.1, 2.4 Hz), 97.7 (dd, *J* = 27.7, 21.9 Hz), 66.7, 50.5 (d, *J* = 3.8 Hz).

Compound **3d**, 4-(2,5-difluorophenyl)morpholine:

Synthesized from 1,2,4-trifluorobenzene according to the general procedure. After 14 hours at 50°C the compound **3d** was isolated in 73% yield as a yellowish solid. $^1H$ NMR ($CDCl_3$) δ ppm 6.96 (ddd, *J* = 12.1, 8.7, 5.2 Hz, 1H), 6.73 - 6.44 (m, 2H), 3.95 -3.73 (m, 4H), 3.16 - 2.89 (m, 4H). $^{13}C$ NMR ($CDCl_3$) δ ppm 159.1 (dd, *J* = 241.9, 2.2 Hz), 151.6 (dd, *J* = 241.2, 2.9 Hz), 141.0 (dd, *J* = 10.5, 8.8 Hz), 116.5 (dd, *J* = 23.6, 10.0 Hz), 107.9 (dd, *J* = 23.9, 8.3 Hz), 105.8 (dd, *J* = 26.3, 3.5 Hz), 66.8, 50.5 (d, *J* = 3.8 Hz).

Compound **3e**, 4-(3,5-difluorophenyl)morpholine:

Synthesized from 1,3,5-trifluorobenzene according to the general procedure. After 14 hours at 50°C the compound **3e** was isolated in 76% yield as a yellowish solid. $^1H$ NMR ($CDCl_3$) δ ppm 6.39 - 6.32 (m, 2H), 6.28 (tt, *J* = 8.8, 2.2 Hz, 1H), 3.86 - 3.79 (m, 4H), 3.16-3.11 (m, 4H). $^{13}C$ NMR ($CDCl_3$) δ ppm 164.0 (dd, *J* = 244.4, 15.8 Hz), 153.3 (t, *J* = 12.2 Hz), 98.3-97.0 (m), 94.5 (t, *J* = 26.1 Hz), 66.5, 48.3.

Compound **3f**, 4-(2-fluorophenyl)morpholine:

Synthesized from 1,2-difluorobenzene according to the general procedure. After 2.5 hours at 100°C the compound **3f** was isolated in 81% yield as a dark yellow oil. $^1H$ NMR ($CDCl_3$) δ ppm 7.10 - 7.00 (m, 2H), 6.98 - 6.91 (m, 2H), 3.89 - 3.83 (m, 4H), 3.10-3.06 (m, 4H). $^{13}C$ NMR ($CDCl_3$) δ ppm 155.7 (d, *J* = 246.1 Hz), 139.9 (d, *J*= 8.4 Hz), 124.4 (d, *J* = 3.6 Hz), 122.6 (d, *J* = 8.1 Hz), 118.6 (d, *J* = 2.9 Hz), 116.1 (d, *J* = 20.6 Hz), 67.0, 50.9 (d, *J* = 3.4 Hz).

Compound **3g**, 4-(3-fluorophenyl)morpholine:

Synthesized from 1,3-difluorobenzene according to the general procedure. After 2.5 hours at 100°C the compound **3g** was isolated in 86% yield as a dark yellow oil. $^1H$ NMR ($CDCl_3$) δ ppm 7.21 (td, *J* = 8.2, 6.9 Hz, 1H), 6.67 (dd, *J* = 8.3, 2.2 Hz, 1H), 6.61 - 6.53 (m, 2H), 3.88 - 3.81 (m, 4H), 3.19 - 3.12 (m, 4H). $^{13}C$ NMR ($CDCl_3$) δ ppm 163.8 (d, *J* = 243.4 Hz), 152.9 (d, *J* = 9.8 Hz), 130.1 (d, *J* = 10.0 Hz), 110.7 (d, *J* = 2.5 Hz), 106.2 (d, *J* = 21.4 Hz), 102.4 (d, *J* = 25.1 Hz), 66.7, 48.8.

Compound **3h**, 4-(2-chlorophenyl)morpholine:

Synthesized from 1-chloro-2-fluorobenzene according to the general procedure. After 1.5 hours at 100°C the compound **3h** was isolated in 72% yield as a yellow oil. $^1H$ NMR ($CDCl_3$) δ ppm 7.37 (dd, *J* = 7.9, 1.5 Hz, 1H), 7.24 (td, *J* = 7.7, 1.6 Hz, 1H), 7.04 (dd, *J* = 8.1, 1.5 Hz, 1H), 6.99 (td, *J* = 7.7, 1.6 Hz, 1H), 3.88 (t, *J* = 4.6 Hz, 4H), 3.06 (t, *J* = 4.6 Hz, 4H). $^{13}C$ NMR ($CDCl_3$) δ ppm 149.0, 130.7, 128.8, 127.6, 123.9, 120.2, 67.1, 51.6.

Compound **3i**, 4-(4-chlorophenyl)morpholine:

Synthesized from 1-chloro-4-fluorobenzene according to the general procedure. After 14 hours at 80°C the compound **3i** was isolated in 53% yield as a white solid. $^1H$ NMR ($CDCl_3$) δ ppm 7.25 - 7.15 (m, 2H), 6.86 - 6.80 (m, 2H), 3.87 - 3.82 (m, 4H), 3.14-3.07 (m, 4H). $^{13}C$ NMR ($CDCl_3$) δ ppm 149.9, 129.0, 124.9, 116.9, 66.8, 49.3.

Compound **3j**, 4-(2-chloro-5-methylphenyl)morpholine:

Synthesized from 1-chloro-2-fluoro-4-methylbenzene according to the general procedure.

After 14 hours at 90°C the compound **3j** was isolated in 77% yield as a clear oil. $^1H$ NMR ($CDCl_3$) δ ppm 7.23 (d, *J* = 8.0 Hz, 1H), 6.84 (d, *J* = 2.0 Hz, 1H), 6.82 - 6.77 (m, 1H), 3.97 - 3.72 (m, 4H), 3.13 - 2.93 (m, 4H), 2.32 (s, 3H). $^{13}C$ NMR ($CDCl_3$) δ ppm 148.6, 137.5, 130.3, 125.5, 124.5, 120.9, 67.1, 51.6, 21.1.

Compound **3l**, 4-(3-methoxyphenyl)morpholine:

Synthesized from 1-fluoro-3-methoxybenzene according to the general procedure. After 14 hours at 90°C the compound **3l** was isolated in 93% yield as a bright yellow oil. $^1H$ NMR ($CDCl_3$) δ ppm 7.24 - 7.16 (m, 1H), 6.54 (ddd, *J* = 8.3, 2.3, 0.9 Hz, 1H), 6.48 - 6.42 (m, 2H), 3.88 - 3.82 (m, 4H), 3.80 (s, 3H), 3.19 - 3.11 (m, 4H). $^{13}C$ NMR ($CDCl_3$) δ ppm 160.6, 152.6, 129.8, 108.4, 104.6, 102.1, 66.8, 55.1, 49.2.

Compound **3m**, 4-([1,1'-biphenyl]-4-yl)morpholine:

Synthesized from 4-fluoro-1,1'-biphenyl according to the general procedure. After 14 hours at 100°C the compound **3m** was isolated in 65% yield as a yellowish solid. $^1H$ NMR ($CDCl_3$) $\delta$ ppm 7.62 - 7.51 (m, 4H), 7.45 - 7.39 (m, 2H), 7.33 - 7.28 (m, 1H), 7.02 - 6.97 (m, 2H), 3.93 - 3.85 (m, 4H), 3.26 - 3.18 (m, 4H). $^{13}C$ NMR ($CDCl_3$) $\delta$ ppm 150.5, 140.8, 132.6, 128.7, 127.8, 126.5, 126.5, 115.7, 66.9, 49.2.

The reactions proved to be selective to fluorine substitution over chlorine-substitution in derivatives containing both, leading to e.g. **3h-3j**. Such structural motifs are in themselves important for the pharmaceutical industry (see examples of pharmaceutically important compounds in figure 1), while the chlorine substituent may also provide a site for further functionalization e.g. through cross coupling reactions. It should be stressed that the benzyne mechanism is ruled out for the here presented reactions as no or negligible regioisomers are observed in NMR spectra of the crude reaction mixtures.

Of special interests were the findings that no additional halogen substituents were required and that even electron-rich fluorobenzene derivatives are feasible reaction partners. Simply adding LiHMDS in THF and fluorobenzene to a vial containing morpholine **1b** provides product **3k** in 82% yield. Employing 3-fluoroanisole under similar conditions forms the product **3l**, which can be isolated in 93% yield. Formation of product **3l** from morpholine have previously (Desmartes 2002, Katoka 2002, Maes 2004, Urgaonkar 2004, Lerma 2005, Shen 2008, Otsuka 2010, Guo 2010, Lu 2011, Jacobsen 2016) been accomplished by employing, nickel, copper, palladium or ruthenium catalysis utilizing 3-halogen-substituted anisole derivatives. Product **3m** further illustrates the complementary properties of these reactions to cross coupling reactions. This product may be synthesized by a cross coupling reaction of 4-chloro-fluorobenzene and phenyl boronic acid followed by the here presented catalyst-free reaction with morpholine, or in the vice versa reaction order through initial generation of **3i** followed by a cross coupling reaction.

**Example 4**

The methods are useful for arylation of a range of secondary amines as seen in figure 3. Both cyclic and acyclic secondary amines can be functionalized as illustrated by formation of products **3n-3ag** in high yields. Thus, most of these compounds were obtained with a 75-80% yield. Moreover, pyrazine and 1-methylpyrazine can be employed as nucleophiles and the resultant products **3q** and **3r** isolated in 62 and 72% yield, respectively. As seen in figure 1 both mono- and diarylated pyrazines are common substructures in pharmaceutical substances. Interestingly, by simply increasing the amount of base and electrophile, the reaction switched from mono- to bis-substitution on pyrazine to afford **3r** or **3s** respectively.

General reaction conditions used in this example: Amine (1.0 mmol), LiHMDS (1.0M in THF, 1.5 mmol) and benzene derivative (1.5 mmol) was mixed and heated.

The structure or the compounds is provided in figure 3.

Compound **3n**, 1-(3-fluorophenyl)pyrrolidine:

Synthesized from pyrrolidine and 1,3-difluorobenzene according to the general procedure.

After 3 hours at 80°C the compound **3n** was isolated in 80% yield yellow oil. $^1H$ NMR ($CDCl_3$) $\delta$ ppm 7.14 (td, $J$ = 8.2, 6.9 Hz, 1H), 6.39-6.28 (m, 2H), 6.24 (dt, $J$ = 12.4, 2.4 Hz, 1H), 3.30 - 3.21 (m, 4H), 2.08 - 1.97 (m, 4H). $^{13}C$ NMR ($CDCl_3$) $\delta$ ppm 164.1 (d, $J$ = 241.6 Hz), 149.5 (d, $J$ = 11.0 Hz), 130.0 (d, $J$ = 10.5 Hz), 107.3 (d, $J$ = 2.1 Hz), 101.7 (d, $J$ = 21.7 Hz), 98.4 (d, $J$ = 25.4 Hz), 47.7, 25.4.

Compound **3o**, N-benzyl-3,5-difluoro-N-methylaniline:

Synthesized from N-methyl-benzylamine and 1,3,5-trifluorobenzene according to the general procedure. After 14 hours at 50°C the compound **3o** was isolated in 79% yield as a dark yellow oil. $^1H$ NMR ($CDCl_3$) $\delta$ ppm 7.36 - 7.32 (m, 2H), 7.30 - 7.23 (m, 1H), 7.22 - 7.14 (m, 2H), 6.28 - 6.17 (m, 2H), 6.14 (tt, $J$ = 9.1, 2.2 Hz, 1H), 4.52 (s, 2H), 3.03 (s, 3H). $^{13}C$ NMR ($CDCl_3$) $\delta$ ppm 164.2 (dd, $J$ = 242.9, 16.3 Hz), 151.6 (t, $J$ = 13.2 Hz), 137.7, 128.7, 127.2, 126.4, 95.2 - 94.6 (m), 91.4 (t, $J$ = 26.3 Hz), 56.2, 38.7.

Compound **3p**, N-benzyl-3-methoxy-N-methylaniline:

Synthesized from N-methyl-benzylamine and 1-fluoro-3-methoxybenzene according to the general procedure. After 14 hours at 90°C the compound **3p** was isolated in 75% yield as a clear oil. $^1H$ NMR ($CDCl_3$) $\delta$ ppm 7.38 - 7.31 (m, 2H), 7.31 - 7.22 (m, 3H), 7.16 (td, $J$ = 8.1, 2.0 Hz, 1H), 6.46 - 6.38 (m, 1H), 6.37 - 6.31 (m, 2H), 4.56 (s, 2H), 3.80 (s, 3H), 3.04 (s, 3H). $^{13}C$ NMR ($CDCl_3$) $\delta$ ppm 160.7, 151.1, 138.9, 129.8, 128.5, 126.8, 126.7, 105.5, 101.3, 98.9, 56.5,

55.0, 38.5.

Compound **3q**, 1-(2-fluorophenyl)-4-methylpiperazine:

Synthesized from N-methyl-piperazine and 1,2-difluorobenzene according to the general procedure. After 3 hours at 80°C the reaction was quenched by the addition of HCl (0.01 M in $H_2O$), the solvent evaporated and the compound redissolved in $CH_3CN:H_2O$ (1:1). The compound **3q** was isolated as the HCl salt by VLC on C18 gel (0 to 50% $CH_3CN$ in 0.01M HCl) in 62% yield as light brown solid. $^1H$ NMR (DMSO-$d_6$) δ ppm 11.37 (s, 1H), 7.24 - 6.95 (m, 4H), 3.51 - 3.40 (m, 4H), 3.25 - 3.12 (m, 4H), 2.78 (d, *J* = 4.8 Hz, 3H). $^{13}C$ NMR (DMSO-$d_6$) δ ppm 154.84 (d, *J* = 244.3 Hz), 138.29 (d, *J* = 8.6 Hz), 124.97 (d, *J* = 3.4 Hz), 123.40 (d, *J* = 7.9 Hz), 119.63 (d, *J* = 2.6 Hz), 116.14 (d, *J* = 20.3 Hz), 52.27, 47.00 (d, *J* = 3.4 Hz), 41.98.

Compound **3r**, 1-(3-methoxyphenyl)piperazine:

Synthesized according to a modified procedure. Piperazine (3.0 mmol), LiHMDS (1.0M in THF, 1.5 mmol) and 1-fluoro-3-methoxybenzene (1.0 mmol) was mixed and heated. After 3 hours at 80°C the reaction was quenched by the addition of HCl (0.01M in $H_2O$), the solvent evaporated and the compound redissolved in $CH_3CN:H_2O$ (1:1). The compound **3r** was isolated as the HCl salt by VLC on C18 gel (0 to 50% $CH_3CN$ in 0.01M HCl) in 72% yield as light brown solid. $^1H$ NMR (DMSO-$d_6$) δ ppm 9.19 (s, 2H), 7.15 (t, *J* = 8.2 Hz, 1H), 6.56 (dd, *J* = 8.2, 2.3 Hz, 1H), 6.51 (t, *J* = 2.4 Hz, 1H), 6.45 (dd, *J* = 8.1, 2.3 Hz, 1H), 3.72 (s, 3H), 3.43 - 3.25 (m, 4H), 3.27 - 3.07 (m, 4H). $^{13}C$ NMR (DMSO-de) δ ppm 160.23, 150.94, 129.91, 108.65, 105.77, 102.45, 55.01, 45.60, 42.29.

Compound **3s**, 1,4-bis(3-fluorophenyl)piperazine:

Synthesized according to a modified procedure. Piperazine (1.0 mmol), LiHMDS (1.0M in THF, 3.0 mmol) and 1,3-difluorobenzene (3.0 mmol) was mixed and heated.
After 3 hours at 80°C the compound **3s** was isolated in 72% yield as a bright yellow solid. $^1H$ NMR ($CDCl_3$) δ ppm 7.23 (td, *J* = 8.2, 6.9 Hz, 2H), 6.73 (ddd, *J* = 8.4, 2.4, 0.8 Hz, 2H), 6.64 (dt, *J* = 12.2, 2.4 Hz, 2H), 6.57 (tdd, *J* = 8.2, 2.4, 0.8 Hz, 2H), 3.34 (s, 8H). $^{13}C$ NMR ($CDCl_3$) δ ppm 163.8 (d, *J* = 243.6 Hz), 152.7 (d, *J* = 9.8 Hz), 130.2 (d, *J* = 9.9 Hz), 111.4 (d, *J* = 2.6 Hz), 106.3 (d, *J* = 21.4 Hz), 103.0 (d, *J* = 25.0 Hz), 48.7.

Compound **3t**, 1-(2,3-dichlorophenyl)piperazine:

Synthesized according to a modified procedure. Piperazine (3.0 mmol), LiHMDS (1.0M in THF, 3.0 mmol) and 2,3-dichlorofluorobenzene (1.0 mmol) was mixed and heated. After 12 hours at 50°C the reaction was quenched by the addition of HCl (0.01M in $H_2O$), the solvent evaporated and the compound redissolved in $CH_3CN:H_2O$ (1:1). The compound **3t** was isolated as the HCl salt by VLC on C18 gel (0 to 50% CH3CN in 0.01M HCl) in 66% yield as white solid.

Employing 1,2,3-trichlorobenzene as electrophile instead of 2,3-dichlorofluorobenzene the same product was obtained in 51% yield after 22 hours at 65°C. $^1H$ NMR (DMSO-$d_6$) δ ppm 9.50 (s, 2H), 7.42 - 7.30 (m, 2H), 7.20 (dd, *J* = 7.2, 2.3 Hz, 1H), 3.22 (s, 8H). $^{13}C$ NMR (DMSO-de) δ ppm 150.00, 132.69, 128.62, 126.15, 125.20, 119.82, 47.78, 42.98.

Compound **3u**, 8-(3-methoxyphenyl)-1,4-dioxa-8-azaspiro[4.5]decane:

Synthesized from 1,4-dioxa-8-azaspiro[4.5]decane and 1-fluoro-3-methoxybenzene according to the general procedure. After 14 hours at 90°C the compound **3u** was isolated in 90% yield as a yellow oil. $^1H$ NMR ($CDCl_3$) δ ppm 7.16 (t, *J* = 8.2 Hz, 1H), 6.56 (ddd, *J* = 8.2, 2.4, 0.8 Hz, 1H), 6.49 (t, *J* = 2.4 Hz, 1H), 6.40 (ddd, *J* = 8.1, 2.4, 0.8 Hz, 1H), 3.98 (s, 4H), 3.79 (s, 3H), 3.39 - 3.27 (m, 4H), 1.88 - 1.77 (m, 4H). $^{13}C$ NMR ($CDCl_3$) δ ppm 160.50, 152.19, 129.66, 109.24, 107.11, 104.04, 102.81, 64.23, 55.05, 47.51, 34.40.

Compound **3v**, 1-(3,5-difluorophenyl)piperidine:

Synthesized from piperidine and 1,3,5-trifluorobenzene according to the general procedure. After 14 hours at 50°C the compound **3v** was isolated in 61% yield as a clear oil. $^1H$ NMR ($CDCl_3$) δ ppm 6.46 - 6.27 (m, 2H), 6.19 (tt, *J* = 8.9, 2.2 Hz, 1H), 3.23 - 3.04 (m, 4H), 1.72 - 1.47 (m, 6H). $^{13}C$ NMR ($CDCl_3$) δ ppm 164.03 (dd, *J* = 243.2, 16.1 Hz), 153.65 (t, *J* = 12.4 Hz), 98.96 - 96.87 (m), 93.12 (t, *J* = 26.2 Hz), 49.40, 25.32, 24.18.

Compound **3w**, 1-(3-methoxyphenyl)-4-phenylpiperazine:

Synthesized from 1-phenylpiperazine and 1-fluoro-3-methoxybenzene according to the general procedure. After 14 hours at 90°C the compound **3w** was isolated in 73% yield as an off-white solid. $^1H$ NMR ($CDCl_3$) δ ppm 7.34 - 7.27 (m, 2H), 7.22 (t, *J* = 8.2 Hz, 1H), 7.02-6.97 (m, 2H), 6.91 (tt, *J* = 7.3, 1.1 Hz, 1H), 6.62 (ddd, *J* = 8.3, 2.4, 0.8 Hz, 1H), 6.54 (t, *J* = 2.4 Hz, 1H), 6.47 (ddd, *J* = 8.2, 2.4, 0.8 Hz, 1H), 3.82 (s, 3H), 3.35 (s, 8H). $^{13}C$ NMR ($CDCl_3$) δ ppm 160.60, 152.60, 151.19, 129.83, 129.15, 120.03, 116.30, 109.04, 104.72, 102.74, 55.19, 49.34.

Compound **3x**, 1-(2-chlorophenyl)-4-phenylpiperazine:

Synthesized from 1-phenylpiperazine and 1-chloro-2-fluorobenzene according to the general procedure. After 2.5 hours at 100°C the compound **3x** was isolated in 50% yield as a white solid. $^1H$ NMR ($CDCl_3$) δ ppm 7.41 (dd, *J* = 8.0, 1.5 Hz, 1H), 7.35 - 7.29 (m, 2H), 7.29 - 7.22 (m, 1H), 7.11 (dd, *J* = 8.0, 1.6 Hz, 1H), 7.05 - 6.98 (m, 3H), 6.91 (ddt, *J* = 8.4, 7.3, 1.1 Hz, 1H), 3.41 - 3.36 (m, 4H), 3.26 - 3.21 (m, 4H). $^{13}C$ NMR ($CDCl_3$) δ ppm 151.35, 149.12, 130.68, 129.12, 128.84, 127.60, 123.84, 120.35, 119.85, 116.21, 51.30, 49.49.

Compound **3y**, N-butyl-N-ethyl-3-methoxyaniline:

Synthesized from N-ethylbutan-1-amine and 1-fluoro-3-methoxybenzene according to the general procedure. After 14 hours at 90°C the compound **3y** was isolated in 82% yield as a clear oil. $^1H$ NMR ($CDCl_3$) δ ppm 7.17 (td, *J* = 8.5, 1.0 Hz, 1H), 6.40-6.31 (m, 1H), 6.33 - 6.19 (m, 2H), 3.40 (q, *J* = 7.1 Hz, 2H), 3.34 - 3.24 (m, 2H), 1.69 - 1.57 (m, 2H), 1.45 - 1.36 (m, 2H), 1.20 (t, *J* = 6.9 Hz, 3H), 1.02 (t, *J* = 7.4 Hz, 3H). $^{13}C$ NMR ($CDCl_3$) δ ppm 160.86, 149.36, 129.80, 105.01, 99.78, 98.32, 55.02, 50.20, 44.96, 29.71, 20.35, 13.99, 12.32.

Compound **3z**, 8-(3,5-difluorophenyl)-1,4-dioxa-8-azaspiro[4.5]decane:

Synthesized from 1,4-dioxa-8-azaspiro[4.5]decane and 1,3,5-trifluorobenzene according to the general procedure. After 14 hours at 50°C the compound **3z** was isolated in 76% yield as an yellow oil. $^1H$ NMR ($CDCl_3$) δ ppm 6.43 - 6.30 (m, 2H), 6.25 - 6.12 (m, 1H), 3.98 (s, 4H), 3.41 - 3.25 (m, 4H), 1.84 - 1.69 (m, 4H). $^{13}C$ NMR ($CDCl_3$) δ ppm 164.02 (dd, *J* = 243.7, 16.1 Hz), 152.48 (t, *J* = 12.4 Hz), 106.89, 99.15-97.06 (m), 93.50 (t, *J* = 26.2 Hz), 64.36, 46.59, 34.08.

Compound **3aa**, 2-(2-chlorophenyl)-1,2,3,4-tetrahydroisoquinoline:

Synthesized from 1,2,3,4-tetrahydroisoquinoline and 1-chloro-2-fluorobenzene according to the general procedure. After 2.5 hours at 100°C the compound **3aa** was isolated in 74% yield as an yellow oil. $^1H$ NMR ($CDCl_3$) δ ppm 7.59 - 7.38 (m, 1H), 7.38 - 7.14 (m, 6H), 7.12 - 6.87 (m, 1H), 4.34 (s, 2H), 3.47 (t, *J* = 5.8 Hz, 2H), 3.10 (t, *J* = 5.9 Hz, 2H). $^{13}C$ NMR ($CDCl_3$) δ ppm 149.17, 134.75, 134.57, 130.76, 129.04, 128.88, 127.56, 126.43, 126.36, 125.84, 123.64, 120.69, 53.32, 49.99, 29.16.

Compound **3ab**, N-butyl-N-ethyl-3,5-difluoroaniline:

Synthesized from N-ethylbutan-1-amine and 1,3,5-trifluorobenzene according to the general procedure. After 14 hours at 50°C the compound **3ab** was isolated in 41% yield as a clear oil. $^1H$ NMR ($CDCl_3$) δ ppm 6.19 - 5.98 (m, 3H), 3.32 (q, *J* = 7.1 Hz, 2H), 3.25-3.14 (m, 2H), 1.63 - 1.49 (m, 2H), 1.42 - 1.31 (m, 2H), 1.15 (t, *J* = 7.1 Hz, 3H), 0.97 (t, *J* = 7.3 Hz, 3H). $^{13}C$ NMR ($CDCl_3$) δ ppm 164.41 (dd, *J* = 241.7, 16.7 Hz), 149.98 (t, *J* = 13.3 Hz), 94.43 - 93.37 (m), 90.09 (t, *J* = 26.5 Hz), 50.28, 45.12, 29.46, 20.26, 13.92, 12.08.

Compound **3ac**, N,N-dibenzyl-3-methoxyaniline:

Synthesized from dibenzylamine and 1-fluoro-3-methoxybenzene according to the general procedure. After 14 hours at 90°C the compound **3ac** was isolated in 89% yield as a clear oil. $^1H$ NMR ($CDCl_3$) δ ppm 7.40 - 7.32 (m, 4H), 7.32 - 7.20 (m, 6H), 7.11 (td, *J* = 8.2, 1.4 Hz, 1H), 6.41 (ddd, *J* = 8.4, 2.3, 1.2 Hz, 1H), 6.36 - 6.28 (m, 2H), 4.67 (s, 4H), 3.76 - 3.68 (m, 3H). $^{13}C$ NMR ($CDCl_3$) δ ppm 160.73, 150.59, 138.50, 129.86, 128.60, 126.85, 126.61, 105.61, 101.49, 99.07, 54.99, 54.20.

Compound **3ad**, N-benzyl-2-chloroaniline:

Synthesized by a modified procedure. Benzyl amine (2.5 mmol), LiHMDS (1.0M in THF, 2.5 mmol) and 2-chlorofluorobenzene (0.5 mmol) were mixed and heated. After 3 hours at 100°C the compound **3ad** was isolated in 42% yield as a clear oil. $^1H$ NMR ($CDCl_3$) $\delta$ ppm 7.26 - 7.21 (m, 4H), 7.20 - 7.15 (m, 2H), 6.98 (td, *J* = 7.8, 1.5 Hz, 1H), 6.56 - 6.49 (m, 2H), 4.64 (d, *J* = 5.7 Hz, 1H), 4.29 (d, *J* = 5.7 Hz, 2H). $^{13}C$ NMR ($CDCl_3$) $\delta$ ppm 143.82, 138.71, 129.07, 128.68, 127.76, 127.31, 127.23, 119.07, 117.38, 111.47, 47.81.

Compound **3ae**, N-benzyl-3,5-difluoroaniline:

Synthesized by a modified procedure. Benzyl amine (2.5 mmol), LiHMDS (1.0M in THF, 2.5 mmol) and 1,3,5-trifluorobenzene (0.5 mmol) were mixed and heated. After 14 hours at 50°C the compound **3ae** was isolated in 41% yield as an yellow oil. $^1H$ NMR ($CDCl_3$) $\delta$ ppm 7.42 - 7.27 (m, 5H), 6.21 - 6.08 (m, 3H), 4.30 (s, 2H), 4.28 (s, 1H). $^{13}C$ NMR ($CDCl_3$) $\delta$ 164.16 (dd, *J* = 244.2, 16.0 Hz), 150.28 (t, *J* = 13.3 Hz), 138.23, 128.82, 127.61, 127.42, 96.68 - 94.46 (m), 92.60 (t, *J* = 26.1 Hz), 48.08.

Compound **3af**, 4-(4-methylpiperazin-1-yl)benzothiophene-2-carboxylic acid: Synthesized by a modified procedure. N-methyl-piperazine (0.2 mmol), 4-fluoro-1-benzothiophene-2-carboxylic acid (0.1 mmol) and LiHMDS (1.0 M in THF, 0.25 mmol) were mixed and heated. After 14 hours at 65°C the reaction was quenched by the addition of HCl (1.0 M in $H_2O$), the solvent evaporated and the compound redissolved in $CH_3CN$:$H_2O$ (1:1). The compound **3af** was isolated as the HCl salt by chromatography on C18 gel (0 to 50% $CH_3CN$ in 0.01 M HCl) in 31% yield as white solid. $^1H$ NMR (500 MHz, DMSO-d6) $\delta$ 13.53 (s, 1H), 10.74 (s, 1H), 8.04 (s, 1H), 7.73 (d, *J*= 8.2 Hz, 1H), 7.46 (t, *J* = 7.9 Hz, 1H), 7.05 (d, *J* = 7.6 Hz, 1H), 3.60 - 3.48 (m, 4H), 3.42 - 3.31 (m, 2H), 3.24 - 3.12 (m, 2H), 2.86 (d, *J* = 4.7 Hz, 3H). $^{13}C$ NMR (126 MHz, DMSO) $\delta$ 163.4, 148.1, 142.9, 133.7, 132.9, 128.0, 127.5, 118.0, 113.6, 52.7, 48.9, 42.1.

Compound **3ag**, 1-methyl-6-(4-phenylpiperazin-1-yl)-indole:

Synthesized by a modified procedure. 1-Phenylpiperazine (0.2 mmol), 6-fluoro-1-methyl-indole (0.1 mmol) and LiHMDS (1.0 M in THF, 0.2 mmol) were mixed and heated. After 14 hours at 90°C in a sealed tube the reaction was quenched by the addition of HCl (1.0 M in $H_2O$), the solvent evaporated and the compound redissolved in $CH_3CN$:$H_2O$ (1:1). The compound 3ag was isolated as the HCl salt by chromatography on C18 gel (0 to 50% $CH_3CN$ in 0.01M HCl) in 53% yield as white solid.
$^1H$ NMR (500 MHz, DMSO-d6) $\delta$ 8.07 (s, 1H), 7.70 (d, *J* = 8.5 Hz, 1H), 7.65 - 7.54 (m, 1H), 7.51 (d, *J* = 3.1 Hz, 1H), 7.40 - 7.24 (m, 3H), 7.18 - 7.07 (m, 2H), 6.98 - 6.90 (m, 1H), 6.52 (d, *J* = 3.2 Hz, 1H), 3.91 -3.67 (m, 11H).$^{13}C$ NMR (126 MHz, DMSO) $\delta$ 149.3, 135.8, 132.0, 129.2, 129.1, 121.3, 120.2, 116.3, 116.1, 112.2, 103.0, 100.7, 54.5, 46.5, 32.7.

**Example 5**

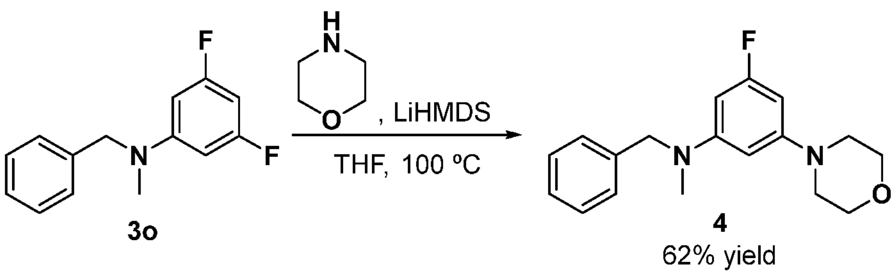


**Scheme 1:** Substitutions on aniline derivative.

Compound **4**, N-benzyl-3-fluoro-N-methyl-5-morpholinoaniline:

N-benzyl-3,5-difluoro-N-methylaniline (**3o**) (0.5 mmol), morpholine (1.0 mmol) and LiHMDS (1.0M in THF, 1.0 mmol) were mixed and heated. After 24 hours at 100°C the compound **4** was isolated in 62% yield as a bright yellow oil. $^1H$ NMR ($CDCl_3$) $\delta$ ppm 7.37 - 7.29 (m, 2H), 7.29 - 7.19 (m, 3H), 6.06 - 5.97 (m, 3H), 4.52 (s, 2H), 3.85 - 3.78 (m, 4H), 3.13 - 3.07 (m, 4H), 3.02 (s, 3H). $^{13}C$ NMR ($CDCl_3$) $\delta$ ppm 164.9 (d, *J* = 238.5 Hz), 153.4 (d, *J* = 12.5 Hz), 151.6 (d, *J* = 13.2 Hz), 138.5, 128.6, 127.0, 126.6, 95.0 (d, *J* = 1.7 Hz), 91.8 (d, *J* = 18.1 Hz), 91.6 (d, *J* = 17.3 Hz), 66.8, 56.5, 49.2, 38.7.

A two steps reaction applying first a weaker nucleophile followed by an amine nucleophile also proved viable (scheme 2). Applying imidazole as the weak azole nucleophile followed by addition of morpholine provided derivative

**7**, which is a substructure in metabotropic glutamate 5 receptor antagonists with nanomolar activity. Thiophenol also proved highly applicable in this approach, illustrated by the synthesis of recently marketed pharmaceutical antidepressant Vortioxetine **9** from 1,2-difluorobenzene. This should be compared to the present industrial production process, which utilizes two subsequent cross-coupling reactions on 2-bromo-iodobenzene.

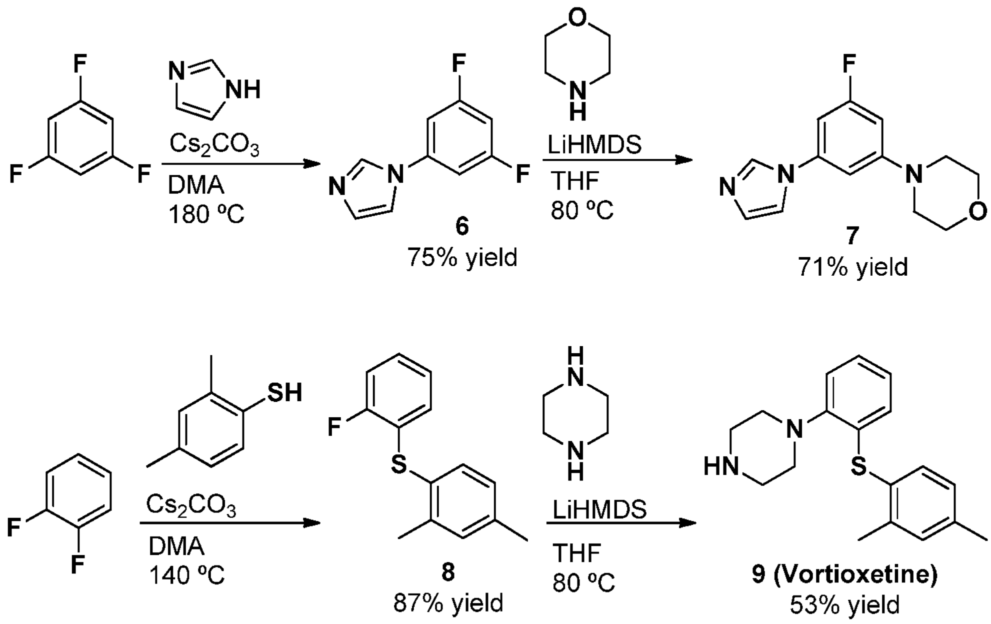


**Scheme 2:** Disubstitution on fluorobenzenes. DMA = dimethylacetamide.

Compound **6**, 1-(3,5-difluorophenyl)-1H-imidazole:

To a vial was added imidazole (1.0 mmol), 1,3,5-trifluorobenzene (2.0 mmol), $Cs_2CO_3$ (3.0 mmol) and dimethylacetamide (2.0 mL). The vial was sealed and stirred at 120 °C for 12 hours. The reaction was quenched with water and brine and then extracted into $Et_2O$. After evaporation, the product **6** was purified by chromatography on silica gel (EtOAc), and isolated in 75% yield as a white solid. $^1H$ NMR ($CDCl_3$) δ ppm 7.90 (t, *J* = 1.1 Hz, 1H), 7.27 (t, *J* = 1.4 Hz, 1H), 7.23 (t, *J* = 1.2 Hz, 1H), 7.01 - 6.93 (m, 2H), 6.83 (tt, *J* = 8.7, 2.3 Hz, 1H). $^{13}C$ NMR ($CDCl_3$) δ ppm 163.6 (dd, *J* = 250.7, 14.1 Hz), 139.2 (t, *J* = 12.2 Hz), 135.3, 130.99, 117.8, 105.5 - 104.1 (m), 102.9 (t, *J* = 25.3 Hz).

Compound **7**, 4-(3-fluoro-5-(1H-imidazol-1-yl)phenyl)morpholine:

Morpholine (0.5 mmol), 1-(3,5-difluorophenyl)-1H-imidazole (**6**) (0.6 mmol) and LiHMDS (1.0M in THF, 1.2 mmol) were mixed and heated. After 2 hours at 80°C the compound **7** was isolated by flash chromatography (5% EtOH in EtOAc) in 71% yield as a yellowish solid. $^1H$ NMR ($CDCl_3$) δ ppm 7.82 (t, *J* = 1.2 Hz, 1H), 7.23 (t, *J* = 1.4 Hz, 1H), 7.18 (d, *J* = 1.2 Hz, 1H), 6.63 (d, *J* = 2.2 Hz, 1H), 6.60-6.51 (m, 2H), 3.91 - 3.81 (m, 4H), 3.26 - 3.15 (m, 4H). $^{13}C$ NMR ($CDCl_3$) δ ppm 164.2 (d, *J* = 245.0 Hz), 153.5 (d, *J* = 11.4 Hz), 139.2 (d, *J* = 13.0 Hz), 135.6, 130.5, 118.3, 103.7 (d, *J* = 2.5 Hz), 100.8 (d, *J* = 25.4 Hz), 99.9 (d, *J* = 25.6 Hz), 66.5, 48.3.

Compound **8**, 2-(2,4-di-methyl-thiophenol-yl)-fluorobenzene:

To a vial was added 2,4-dimethylthiophenol (1.0 mmol), 1,2-difluorobenzene (2.0 mmol), $Cs_2CO_3$ (2.5 mmol) and dimethylacetamide (2.0 mL). The vial was sealed and stirred at 140 °C for 4 hours. The reaction was quenched with water and brine and then extracted into $Et_2O$. After evaporation, the product **8** was purified by VLC on silica gel, and isolated in 87% yield as a clear oil. $^1H$ NMR ($CDCl_3$) δ ppm 7.28 (d, *J* = 7.8 Hz, 1H), 7.19 - 7.09 (m, 2H), 7.09 - 7.03 (m, 1H), 7.02 - 6.95 (m, 2H), 6.87 (td, *J* = 7.7, 1.7 Hz, 1H), 2.37 (s, 3H), 2.34 (s, 3H). $^{13}C$ NMR ($CDCl_3$) δ ppm 160.0 (d, *J* = 245.2 Hz), 141.0, 138.9, 134.4, 131.7, 130.2 (d, *J* = 1.9 Hz), 127.7, 127.6, 127.5 (d, *J* = 7.5 Hz), 124.6 (d, *J* = 3.5 Hz), 124.4, 115.5 (d, *J* = 21.8 Hz), 21.1, 20.5.

Compound **9**, 1-[2-(2,4-Dimethyl-phenylsulfanyl)-phenyl]piperazine:

Piperazine (1.25 mmol), 2-(2,4-di-methyl-thiophenol-yl)-fluorobenzene (**8**) (0.25 mmol) and LiHMDS (1.0 M in THF, 1.0 mmol) were mixed and heated in a sealed vial. After 2 hours at 80°C the reaction was quenched by the addition of HCl (0.01M in $H_2O$), the solvent evaporated and the compound redissolved in $CH_3CN$:$H_2O$ (1:1). The compound **9** was isolated as the HCl salt by VLC on C18 gel (0 to 50% $CH_3CN$ in 0.01M HCl) in 53% yield as white solid. $^1H$ NMR (DMSO-$d_6$) $\delta$ ppm 9.41 (s, 2H), 7.33 (d, *J* = 7.8 Hz, 1H), 7.24 (d, *J* = 1.9 Hz, 1H), 7.16 - 7.07 (m, 3H), 6.96 (ddd, *J* = 7.9, 5.9, 2.7 Hz, 1H), 6.44 - 6.39 (m, 1H), 3.21 (s, 8H), 2.32 (s, 3H), 2.24 (s, 3H). $^{13}C$ NMR (DMSO-$d_6$) $\delta$ 147.8, 141.6, 139.3, 135.7, 133.3, 131.7, 128.1, 126.8, 126.0, 125.8, 125.1, 120.2, 48.1, 43.3, 20.7, 20.1.

The disclosure provides a novel method for the amination of unactivated fluorobenzene derivatives. A key factor for reactivity is the applied base's ability to sufficiently deprotonate the amine nucleophile under the applied reaction conditions without simultaneously degrading the fluorobenzene electrophile. For secondary aliphatic amines the reactions proceed readily by addition of a simple base such as LiHMDS, and thus circumvent the need for transition metals. The reactions proceed with great regio- and chemoselectivity and are compatible with a broad range of additional substituents including alkyl, aryl, alkoxy, amine, azolyl, thioethers, fluorine and chlorine. The versatility of these new reactions was illustrated by the synthesis of a variety of anilinesincluding the antidepressant Vortioxetine.

## References

Hansch et al. Chem. Rev. 1991, 91, 165-195

C. Desmarets, R. Schneider, Y. Fort, J. Org. Chem. 2002, 67, 3029-3036

N. Kataoka, Q. Shelby, J. P. Stambuli, J. F. Hartwig, J. Org. Chem. 2002, 67, 5553-5566

B. U. Maes, K. T. Loones, S. Hostyn, G. Diels, G. Rombouts, Tetrahedron 2004, 60, 11559-11564

S. Urgaonkar, J. G. Verkade, J. Org. Chem. 2004, 69, 9135-9142

I. C. Lerma, M. J. Cawley, F. G. Cloke, K. Arentsen, J. S. Scott, S. E. Pearson, J. Hayler, S. Caddick, J. Organomet. Chem. 2005, 690, 5841-5848

Q. Shen, T. Ogata, J. F. Hartwig, J. Am. Chem. Soc. 2008, 130, 6586-6596 M. Otsuka, K. Endo, T. Shibata, Chem. Comm. 2010, 46, 336-338

D. Guo, H. Huang, Y. Zhou, J. Xu, H. Jiang, K. Chen, H. Liu, Green Chem. 2010, 12, 276-281

B. Lü, P. Li, C. Fu, L. Xue, Z. Lin, S. Ma, Adv. Synt. Cat. 2011, 353, 100-112. Jacobsen, C. B.; Meldal, M.; Diness, F.Chemistry - A European Journal, 2016, accepted, DOI: 10.1002/chem.201604098


## Claims

1. A method for preparing an arylated amine or a salt thereof, said method comprising the steps of

    a. Providing a nucleophile, wherein said nucleophile comprises an -NH- or an -$NH_2$ group directly linked to only non-aromatic carbon atoms or a salt of said nucleophile;
    b. Providing an electrophile, wherein said electrophile is aryl substituted with at least two substituents, wherein the first substituent is halogen and the second substituent and any further optional substituent(s) are selected from the group consisting of halogen, aryl, substituted aryl, alkenyl, substituted alkenyl, heteroalkenyl, alkyl, substituted alkyl, heteroalkyl, alkoxy, substituted alkoxy, amino, thioalkyl, substituted thioalkyl, thioaryl, substituted thioaryl, heteroaryl, and phosphinyl, with the proviso that said aryl is substituted with at the most 4 halogens; wherein two of said substituents may be fused to form a ring.
    c. Providing a base, wherein the corresponding acid has a pKa above 29 in DMSO and/or a pKa above 25 in THF;
    d. Providing an organic solvent that only contain protons with a pKa above 32 in DMSO;
    e. Reacting said nucleophile with said electrophile in said organic solvent in the presence of the base, thereby obtaining an arylated amine consisting of said aryl, wherein the first substituent is substituted by said amine, wherein this is performed in the absence of a transition metal catalyst;
    f. Optionally purifying the arylated amine or a salt thereof,
    wherein steps a., b., c. and d. may be performed in any order.

2. The method according to claim 1, wherein step b. consists of providing an electrophile, wherein said electrophile is aryl substituted with at least two substituents, wherein the first substituent is halogen and the second substituent and any further optional substituent(s) are selected from the group consisting of halogen, aryl, substituted aryl, alkyl,

substituted alkyl, alkoxy, substituted alkoxy, amino, substituted amino, thioalkyl, substituted thioalkyl, thioaryl, substituted thioaryl, heteroaryl, and phosphinyl, with the proviso that said aryl is substituted with at the most 4 halogens.

**3.** The method according to any one of the preceding claims, wherein the nucleophile is a compound of the formula I:

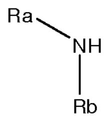 (I),

wherein

$R_a$ and $R_b$ individually are selected from the group consisting of -H and alkyl, wherein said alkyl optionally may be substituted with aryl or substituted aryl with the proviso that only one of $R_a$ and $R_b$ may be -H; or
$R_a$ and $R_b$ together forms a non-aromatic heterocycle, which comprises one or more heteroatoms,
or a salt thereof.

**4.** The method according to claim 3, wherein $R_a$ and $R_b$ individually are selected from the group consisting of alkyl, wherein said alkyl optionally may be substituted with aryl; or
$R_a$ and $R_b$ together forms a non-aromatic heterocycle, which optionally may comprise one or more heteroatoms; such as a 4 to 10 membered non-aromatic heterocycle comprising at least one N atom; or a salt thereof.

**5.** The method according to any one of the preceding claims, wherein the nucleophile is a compound of the formula II

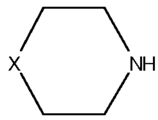 (II)

wherein X is $NR_c$, NH, O or S, and wherein $R_c$ is aryl, substituted aryl, heteroaryl, substistuted heteroaryl, alkyl, substituted alkyl, alkenyl, substituted alkenyl, alkynyl, substituted alkynyl, 3 to 8 membered cycloalkyl or 3-8 membered nonaromatic heterocycle.

**6.** The method according to any one of the preceding claims, wherein the nucleophile is selected from the group consisting of N-methylpiperazine, morpholine, pyrrolidine, N-methyl-benzylamine, piperazine, 1,4-dioxa-8-azaspiro[4.5]decane, piperidine, 1-phenylpiperazine, N-ethylbutan-1-amine, 1,4-dioxa-8-azaspiro[4.5]decane, 1,2,3,4-tetrahydroisoquinoline and dibenzylamine, benzyl amine or salts hereof.

**7.** The method according to any one of the preceding claims, wherein said electrophile is selected from the group consisting of benzene, arene, indole, benzothiophene, benzofuran, benzimidazole, benzoxazole, benzothiazole, indazole, benzotriazole, isoindoline and indoline (shown below), substituted with at least one F or Cl, optionally further substituents selected from the group of consisting of aryl,

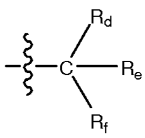 ,

$R_gO$-,

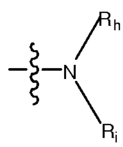 ,

Azolyl- Br-, Cl-, F-, $F_3C$, $R_jS$-, and

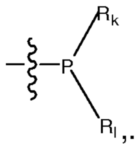

wherein $R_d$, $R_e$, $R_f$, $R_g$, $R_h$, $R_i$, $R_j$, $R_k$ and $R_l$ individually are selected from the group consisting of -H, aryl, substituted aryl, heteroaryl, substituted heteroaryl, alkyl, substituted alkyl, alkenyl, substituted alkenyl, alkynyl and substituted alkynyl.

**8.** The method according to any one of the preceding claims, wherein the electrophile is benzene substituted with 1 -F, a $2^{nd}$ substitutent and optionally a $3^{rd}$, $4^{th}$, $5^{th}$ and/or $6^{th}$ substituent, wherein said $2^{nd}$, $3^{rd}$, $4^{th}$, $5^{th}$ and/or $6^{th}$ substituent individually are selected from the group consisting of -Cl, aryl, substituted aryl, alkenyl, substituted alkenyl, heteroalkenyl, alkyl, substituted alkyl, heteroalkyl, alkoxy, substituted alkoxy, amino, thioalkyl, substituted thioalkyl, heteroaryl, and phosphinyl.

**9.** The method according to any one of the preceding claims, wherein the electrophile is selected from the group consisting of 2,3-dichloro-fluorobenzene, 1,2,3-trichlorobenzene, 2-(2,4-di-methyl-thiophenol-yl)-fluorobenzene, 2-(2,4-di-methyl-thiophenol-yl)-chlorobenzene, 1,3,5-trifluorobenzene, 1,2,3,5-tetrafluorobenzene, 1,2,4-trifluorobenzene, 1,2-difluorobenzene, 1,3-difluorobenzene, 1-chloro-2-fluorobenzene, 1-chloro-4-fluorobenzene, 1-chloro-2-fluoro-4-methylbenzene, 1-fluoro-3-methoxybenzene, 4-fluoro-1,1'-biphenyl, 2,3-dichlorofluorobenzene, 2-chlorofluorobenzene, 4-fluoro-1-benzothiophene-2-carboxylic acid, 6-fluoro-1-methyl-indole, N-benzyl-3,5-difluoro-N-methylaniline, and 1-(3,5-difluorophenyl)-1H-imidazole.

**10.** The method according to any one of the preceding claims, wherein the solvent is a solvent that only contains protons with a pKa above 35.1 in DMSO, and contains no sulfoxide groups.

**11.** The method according to any one of the preceding claims, wherein the solvent is selected from the group consisting of ethers, alkanes, benzene and substituted benzene; and contains no carbonyl groups.

**12.** The method according to any one of the preceding claims, wherein the solvent is selected from the group consisting of tetrahydrofuran (THF), dioxane, dimethoxyethane (DME), 2-methyl-tetrahydrofuran (2-Me-THF), methylcyclohexane, xylene, toluene and chlorobenzene.

**13.** The method according to any one of the preceding claims, wherein the base has a pKa higher than 29 in DMSO and/or a pKa higher than 25 in THF, and/or a pKa of less than 45 in THF; and a $M_w$ of at least 120 g/mol, such as at least 140 g/mol.

**14.** The method according to any one of the preceding claims, wherein the base is selected from the group consisting of LiHMDS, NaHMDS, KHMDS and LiTMP.

**15.** The method according to any one of the preceding claims, wherein the reaction in step e. is performed at a temperature of at the most 120 °C, such as at the most 110°C and takes places for in the range of 5 min to one week, such as 5 min to 900 min.

**Patentansprüche**

**1.** Verfahren zur Herstellung eines arylierten Amins oder eines Salzes davon, wobei das Verfahren folgende Schritte umfasst:

a. Bereitstellen eines Nucleophils, wobei das Nucleophil eine -NH-- oder eine -$NH_2$-Gruppe umfasst, die nur mit nichtaromatischen Kohlenstoffatomen oder einem Salz des Nucleophils direkt verbunden ist;
b. Bereitstellen eines Elektrophils, wobei das Elektrophil ein Aryl ist, das mit mindestens zwei Substituenten substituiert ist, wobei der erste Substituent Halogen ist und der zweite Substituent und alle weiteren optionalen Substituenten aus der Gruppe ausgewählt sind, die aus Halogen, Aryl, substituiertem Aryl, Alkenyl, substituiertem Alkenyl, Heteroalkenyl, Alkyl, substituiertem Alkyl, Heteroalkyl, Alkoxy, substituiertem Alkoxy, einer Aminoverbindung, Thioalkyl, substituiertem Thioalkyl, Thioaryl, substituiertem Thioaryl, Heteroaryl und Phosphinyl besteht, unter der Voraussetzung, dass das Aryl mit höchstens 4 Halogenen substituiert ist; wobei zwei der Substituenten zu einem Ring verbunden sein können.
c. Bereitstellen einer Base, wobei die entsprechende Säure einen $pK_S$-Wert aufweist, der in DMSO über 29 liegt, oder einen $pK_S$-Wert aufweist, der in THF über 25 liegt;
d. Bereitstellen eines organischen Lösungsmittels, das nur Protonen enthält, deren $pK_S$-Wert in DMSO über 32 liegt;
e. Reagierenlassen des Nucleophils mit dem Elektrophil in dem organischen Lösungsmittel in Anwesenheit der Base, wodurch ein aryliertes Amin erhalten wird, das aus dem Aryl besteht, wobei der erste Substituent durch das Amin substituiert ist, wobei dies in Abwesenheit eines Übergangsmetallkatalysators durchgeführt wird;
f. Optional Reinigen des arylierten Amins oder eines Salzes davon, wobei die Schritte a., b., c. und d. in beliebiger Reihenfolge durchgeführt werden können.

2. Verfahren nach Anspruch 1, wobei Schritt b. daraus besteht, ein Elektrophil bereitzustellen, wobei das Elektrophil Aryl ist, das mit mindestens zwei Substituenten substituiert ist, wobei der erste Substituent Halogen ist und der zweite Substituent und alle weiteren optionalen Substituenten aus der Gruppe ausgewählt sind, die aus Halogen, Aryl, substituiertem Aryl, Alkoxy, substituiertem Alkoxy, einer Aminoverbindung, einer substituierten Aminoverbindung, Thioalkyl, substituiertem Thioalkyl, Thioaryl, substituiertem Thioaryl, Heteroaryl und Phosphinyl besteht, unter der Voraussetzung, dass das Aryl mit höchstens 4 Halogenen substituiert ist.

3. Verfahren nach einem oder mehreren der vorhergehenden Ansprüche, wobei das Nucleophil eine Verbindung der Formel I ist:

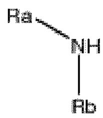
(I),

wobei

$R_a$ und $R_b$ individuell aus der Gruppe ausgewählt sind, die aus -H und Alkyl besteht, wobei das Alkyl optional mit Aryl oder substituiertem Aryl substituiert werden kann, unter der Voraussetzung, dass nur eines von $R_a$ und $R_b$ -H sein kann; oder
$R_a$ und $R_b$ zusammen einen nichtaromatischen Heterocyclus bilden, der ein oder mehrere Heteroatome umfasst, oder ein Salz davon.

4. Verfahren nach Anspruch 3, wobei $R_a$ und $R_b$ individuell aus der Gruppe ausgewählt sind, die aus Alkyl besteht, wobei das Alkyl optional mit Aryl substituiert werden kann; oder
$R_a$ und $R_b$ zusammen einen nichtaromatischen Heterocyclus bilden, der optional ein oder mehrere Heteroatome umfassen kann; sodass ein 4- bis 10-gliedriger, nichtaromatischer Heterocyclus mindestens ein N-Atom umfasst; oder ein Salz davon.

5. Verfahren nach einem der vorhergehenden Ansprüche, wobei das Nucleophil eine Verbindung der Formel II ist:

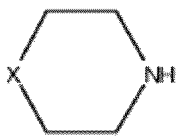
(II)

wobei X $NR_C$, NH, O oder S ist und wobei $R_C$ Aryl, substituiertes Aryl, Heteroaryl, substituiertes Heteroaryl, Alkyl, substituiertes Alkyl, Alkenyl, substituiertes Alkenyl, Alkynyl, substituiertes Alkynyl, ein 3- bis 8-gliedriges Cycloalkyl oder ein 3- bis 8-gliedriger, nichtaromatischer Heterocyclus ist.

6. Verfahren nach einem der vorhergehenden Ansprüche, wobei das Nucleophil aus der Gruppe ausgewählt ist, die aus N-Methylpiperazin, Morpholin, Pyrrolidin, N-Methylbenzylamin, Piperazin, 1,4-Dioxa-8-azaspiro[4.5]decan, Piperidin, 1-Phenylpiperazin, N-Ethylbutan-1-amin, 1,4-Dioxa-8-azaspiro[4.5]decan, 1,2,3,4-Tetrahydroisoquinolin

und Dibenzylamin, Benzylamin oder Salzen davon besteht.

**7.** Verfahren nach einem der vorhergehenden Ansprüche, wobei das Elektrophil aus der Gruppe ausgewählt ist, die aus Benzol, Aren, Indol, Benzothiophen, Benzofuran, Benzimidazol, Benzoxazol, Benzothiazol, Indazol, Benzotriazol, Isoindolin und Indolin (siehe unten) besteht, substituiert mit mindestens einem F oder Cl, optional mit weiteren Substituenten, die aus der Gruppe ausgewählt sind, die aus Aryl,

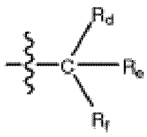

,

$R_gO$-,

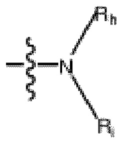

,

Azolyl- Br-, Cl-, F-, $F_3C$, $R_jS$- und

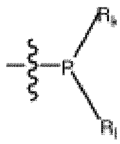

besteht,
wobei $R_d$, $R_e$, $R_f$, $R_g$, $R_h$, $R_i$, $R_j$, $R_k$ und $R_l$ individuell aus der Gruppe ausgewählt sind, die aus -H, Aryl, substituiertem Aryl, Heteroaryl, substituiertem Heteroaryl, Alkyl, substituiertem Alkyl, Alkenyl, substituiertem Alkenyl, Alkynyl und substituiertem Alkynyl besteht.

**8.** Verfahren nach einem der vorhergehenden Ansprüche, wobei das Elektrophil Benzol ist, das mit 1-F, einem 2. Substituenten und wahlweise einem 3., 4., 5. und/oder 6. Substituenten substituiert ist, wobei der 2., 3., 4., 5. und/oder 6. Substituent individuell aus der Gruppe ausgewählt sind, die aus -Cl, Aryl, substituiertem Aryl, Alkenyl, substituiertem Alkenyl, Heteroalkenyl, Alkyl, substituiertem Alkyl, Heteroalkyl, Alkoxy, substituiertem Alkoxy, einer Aminoverbindung, Thioalkyl, substituiertem Thioalkyl, Heteroaryl und Phosphinyl besteht.

**9.** Verfahren nach einem der vorhergehenden Ansprüche, wobei das Elektrophil aus der Gruppe ausgewählt ist, die aus 2,3-Dichlorfluorbenzol, 1,2,3-Trichlorbenzol, 2-(2,4-Dimethylthiophenolyl)-fluorbenzol, 2-(2,4-Dimethylthiophenolyl)-chlorbenzol, 1,3,5-Trifluorbenzol, 1,2,3,5-Tetrafluorbenzol, 1,2,4-Trifluorbenzol, 1,2-Difluorbenzol, 1,3-Difluorbenzol, 1-Chlor-2-fluorbenzol, 1-Chlor-4-Fluorbenzol, 1-Chlor-2-fluor-4-metyhlbenzol, 1-Fluor-3-methoxybenzol, 4-Fluor-1,1'-biphenyl, 2,3-Dichlorfluorbenzol, 2-Chlorofluorbenzol, 4-Fluor-1-benzothiophen-2-carboxylsäure, 6-Fluor-1-methylindol, N-Benzyl-3,5-difluor-N-methylanilin und 1-(3,5-Difluorphenyl)-1H-imidazol besteht.

**10.** Verfahren nach einem der vorhergehenden Ansprüche, wobei das Lösungsmittel ein Lösungsmittel ist, das nur Protonen enthält, deren $pK_s$-Wert in DMSO über 35,1 liegt, und das keine Sulfoxidgruppen enthält.

**11.** Verfahren nach einem der vorhergehenden Ansprüche, wobei das Lösungsmittel aus der Gruppe ausgewählt ist, die aus Ethern, Alkanen, Benzol und substituiertem Benzol besteht; und das keine Carbonylgruppen enthält.

**12.** Verfahren nach einem der vorhergehenden Ansprüche, wobei das Lösungsmittel aus der Gruppe ausgewählt ist, die aus Tetrahydrofuran (THF), Dioxan, Dimethoxyethan (DME), 2-Methlytetrahydrofuran (2-Me-THF), Methylcyclohexan, Xylen, Toluen und Chlorbenzol besteht.

**13.** Verfahren nach einem der vorhergehenden Ansprüche, wobei die Base einen $pK_s$-Wert aufweist, der in DMSO über 29 liegt, und/oder einen $pK_s$-Wert aufweist, der in THF unter 25 liegt, und/oder einen $pK_s$-Wert aufweist, der in THF unter 45 liegt; und eine $M_w$ von mindestens 120 g/mol aufweist, sowie eine $M_w$ von mindestens 140 g/mol.

**14.** Verfahren nach einem der vorhergehenden Ansprüche, wobei die Base aus der Gruppe ausgewählt ist, die aus LiHMDS, NaHMDS, KHMDS und LiTMP besteht.

**15.** Verfahren nach einem der vorhergehenden Ansprüche, wobei die Reaktion in Schritt e. bei einer Temperatur von höchstens 120 °C sowie höchstens 110 °C durchgeführt wird und im Bereich von fünf Minuten bis zu einer Woche sowie von fünf Minuten bis zu 900 Minuten andauert.

**Revendications**

**1.** Procédé de préparation d'une amine arylée ou d'un sel de celle-ci, ledit procédé comprenant les étapes

a. de fourniture d'un nucléophile, dans lequel ledit nucléophile comprend un -NH- ou un groupe $-NH_2$ directement lié à uniquement des atomes de carbone non aromatiques ou à un sel dudit nucléophile ;
b. de fourniture d'un électrophile, dans lequel ledit électrophile est un aryle substitué par au moins deux substituants, dans lequel le premier substituant est un halogène et le second substituant et tout(s) autre(s) substituant(s) éventuel(s) est/sont choisi(s) dans le groupe constitué par un halogène, un aryle, un aryle substitué, un alcényle, un alcényle substitué, un hétéroalcényle, un alkyle, un alkyle substitué, un hétéroalkyle, un alcoxy, un alcoxy substitué, un amino, un thioalkyle, un thioalkyle substitué, un thioaryle, un thioaryle substitué, un hétéroaryle et un phosphinyle, à condition que ledit aryle soit substitué par au plus 4 halogènes ; dans lequel deux desdits substituants peuvent être fusionnés pour former un cycle ;
c. de fourniture d'une base, dans laquelle l'acide correspondant a un pKa supérieur à 29 dans le DMSO et/ou un pKa supérieur à 25 dans le THF ;
d. de fourniture d'un solvant organique qui ne contient que des protons avec un pKa supérieur à 32 dans le DMSO ;
e. de réaction dudit nucléophile avec ledit électrophile dans ledit solvant organique en présence de la base, obtenant ainsi une amine arylée constituée dudit aryle, dans lequel le premier substituant est substitué par ladite amine, dans lequel ceci est effectué en l'absence d'un catalyseur de métal de transition ;
f. de purification éventuelle de l'amine arylée ou d'un sel de celle-ci,
dans lequel les étapes a., b., c. et d. peuvent être exécutées dans n'importe quel ordre.

**2.** Procédé selon la revendication 1, dans lequel l'étape b. consiste à fournir un électrophile, dans lequel ledit électrophile est un aryle substitué par au moins deux substituants, dans lequel le premier substituant est un halogène et le second substituant et tout(s) autre(s) substituant(s) éventuel(s) est/sont choisi(s) dans le groupe constitué par un halogène, un aryle, un aryle substitué, un alkyle, un alkyle substitué, un alcoxy, un alcoxy substitué, un amino, un amino substitué, un thioalkyle, un thioalkyle substitué, un thioaryle, un thioaryle substitué, un hétéroaryle et un phosphinyle, à condition que ledit aryle soit substitué par au plus 4 halogènes.

**3.** Procédé selon l'une quelconque des revendications précédentes, dans lequel le nucléophile est un composé de formule I :

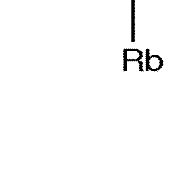
(I),

dans laquelle

$R_a$ et $R_b$ individuellement sont choisis dans le groupe constitué par -H et un alkyle, dans lequel ledit alkyle peut éventuellement être substitué par un aryle ou un aryle substitué à condition qu'un seul parmi $R_a$ et $R_b$ puisse être -H ; ou

$R_a$ et $R_b$ forment ensemble un hétérocycle non aromatique, qui comprend un ou plusieurs hétéroatomes, ou un sel de ceux-ci.

4. Procédé selon la revendication 3, dans lequel $R_a$ et $R_b$ individuellement sont choisis dans le groupe constitué par un alkyle, dans lequel ledit alkyle peut éventuellement être substitué par aryle ; ou
$R_a$ et $R_b$ forment ensemble un hétérocycle non aromatique, qui peut éventuellement comprendre un ou plusieurs hétéroatomes ; tel qu'un hétérocycle non aromatique de 4 à 10 chaînons comprenant au moins un atome N ; ou un sel de celui-ci.

5. Procédé selon l'une quelconque des revendications précédentes, dans lequel le nucléophile est un composé de formule II

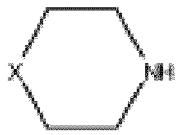
(II)

dans laquelle X est $NR_c$, NH, O ou S, et dans laquelle $R_c$ est un aryle, un aryle substitué, un hétéroaryle, un hétéroaryle substitué, un alkyle, un alkyle substitué, un alcényle, un alcényle substitué, un alcynyle, un alcynyle substitué, un cycloalkyle de 3 à 8 chaînons ou un hétérocycle non aromatique de 3 à 8 chaînons.

6. Procédé selon l'une quelconque des revendications précédentes, dans lequel le nucléophile est choisi dans le groupe constitué par la N-méthylpipérazine, la morpholine, la pyrrolidine, la N-méthyl-benzylamine, la pipérazine, le 1,4-dioxa-8-azaspiro[4,5]décane, la pipéridine, la 1-phénylpipérazine, la N-éthylbutan-1-amine, le 1,4-dioxa-8-azaspiro[4,5]décane, la 1,2,3,4-tétrahydroisoquinoléine et la dibenzylamine, la benzylamine ou leurs sels.

7. Procédé selon l'une quelconque des revendications précédentes, dans lequel ledit électrophile est choisi dans le groupe constitué par le benzène, l'arène, l'indole, le benzothiophène, le benzofurane, le benzimidazole, le benzoxazole, le benzothiazole, l'indazole, le benzotriazole, l'isoindoline et l'indoline (illustré ci-dessous), substitué par au moins un F ou Cl, éventuellement d'autres substituants choisis dans le groupe constitué par un aryle,

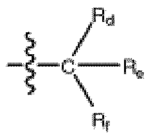
,

$R_gO$-,

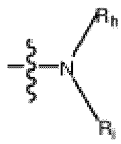

Azolyl- Br-, Cl-, F-, $F_3C$, $R_jS$- et

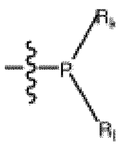

dans laquelle $R_d$, $R_e$, $R_f$, $R_g$, $R_h$, $R_i$, $R_j$, $R_k$ et $R_l$ individuellement sont choisis dans le groupe constitué par -H, un aryle, un aryle substitué, un hétéroaryle, un hétéroaryle substitué, un alkyle, un alkyle substitué, un alcényle, un alcényle substitué, un alcynyle et un alcynyle substitué.

**8.** Procédé selon l'une quelconque des revendications précédentes, dans lequel l'électrophile est le benzène substitué par 1-F, un $2^{ème}$ substituant et éventuellement un $3^{ème}$, $4^{ème}$, $5^{ème}$ et/ou $6^{ème}$ substituant, dans lequel lesdits $2^{ème}$, $3^{ème}$, $4^{ème}$, $5^{ème}$ et/ou $6^{ème}$ substituant individuellement sont choisis dans le groupe constitué par -Cl, un aryle, un aryle substitué, un alcényle, un alcényle substitué, un hétéroalcényle, un alkyle, un alkyle substitué, un hétéroalkyle, un alcoxy, un alcoxy substitué, un amino, un thioalkyle, un thioalkyle substitué, un hétéroaryle et un phosphinyle.

**9.** Procédé selon l'une quelconque des revendications précédentes, dans lequel l'électrophile est choisi dans le groupe constitué par le 2,3-dichloro-fluorobenzène, le 1,2,3-trichlorobenzène, le 2-(2,4-di-méthyl-thiophénol-yl)-fluorobenzène, le 2-(2,4-di-méthyl-thiophénol-yl)-chlorobenzène, le 1,3,5-trifluorobenzène, le 1,2,3,5-tétrafluorobenzène, le 1,2,4-trifluorobenzène, le 1,2-difluorobenzène, le 1,3-difluorobenzène, le 1-chloro-2-fluorobenzène, le 1-chloro-4-fluorobenzène, le 1-chloro-2-fluoro-4-méthylbenzène, le 1-fluoro-3-méthoxybenzène, le 4-fluoro-1,1'-biphényle, le 2,3-dichlorofluorobenzène, le 2-chlorofluorobenzène, l'acide 4-fluoro-1-benzothiophène-2-carboxylique, le 6-fluoro-1-méthyl-indole, la N-benzyl-3,5-difluoro-N-méthylaniline et le 1-(3,5-difluorophényl)-1H-imidazole.

**10.** Procédé selon l'une quelconque des revendications précédentes, dans lequel le solvant est un solvant qui ne contient que des protons avec un pKa supérieur à 35,1 dans le DMSO, et ne contient pas de groupes sulfoxyde.

**11.** Procédé selon l'une quelconque des revendications précédentes, dans lequel le solvant est choisi dans le groupe constitué par des éthers, des alcanes, un benzène et un benzène substitué ; et ne contient aucun groupe carbonyle.

**12.** Procédé selon l'une quelconque des revendications précédentes, dans lequel le solvant est choisi dans le groupe constitué par le tétrahydrofurane (THF), le dioxane, le diméthoxyéthane (DME), le 2-méthyl-tétrahydrofurane (2-Me-THF), le méthylcyclohexane, le xylène, le toluène et le chlorobenzène.

**13.** Procédé selon l'une quelconque des revendications précédentes, dans lequel la base a un pKa supérieur à 29 dans le DMSO et/ou un pKa supérieur à 25 dans le THF, et/ou un pKa inférieur à 45 dans le THF ; et un $M_w$ d'au moins 120 g/mol, tel qu'au moins 140 g/mol.

**14.** Procédé selon l'une quelconque des revendications précédentes, dans lequel la base est choisie dans le groupe constitué par LiHMDS, NaHMDS, KHMDS et LiTMP.

**15.** Procédé selon l'une quelconque des revendications précédentes, dans lequel la réaction de l'étape e. est effectuée à une température d'au plus 120 °C, telle qu'au plus 110 °C et se déroule pendant une durée de 5 minutes à une semaine, telle que de 5 minutes à 900 minutes.

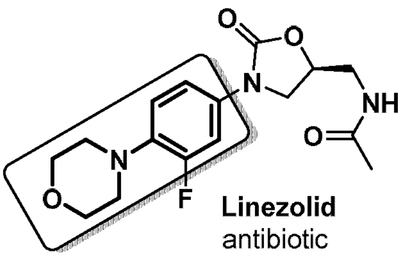

**Linezolid**
antibiotic

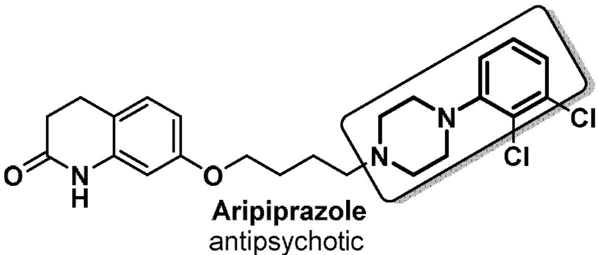

**Aripiprazole**
antipsychotic

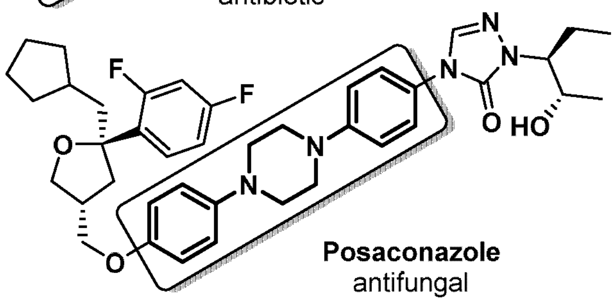

**Posaconazole**
antifungal

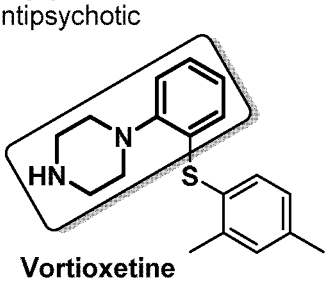

**Vortioxetine**
antidepressant

# Fig. 1

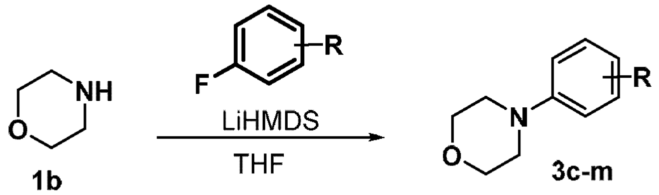


1b 3c-m

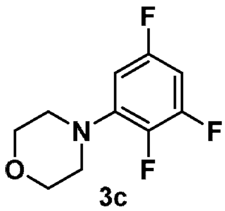

**3c**
70% yield

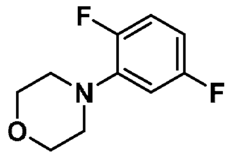

**3d**
73% yield

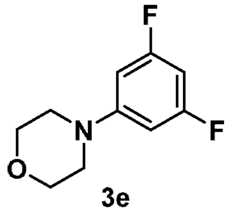

**3e**
76% yield

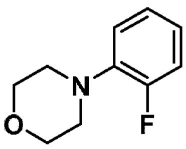

**3f**
81% yield

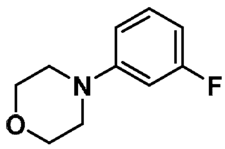

**3g**
86% yield

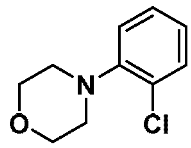

**3h**
72% yield

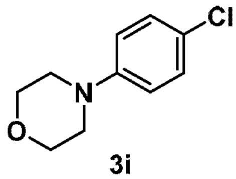

**3i**
53% yield

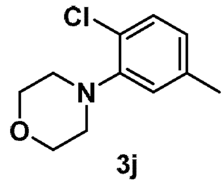

**3j**
77% yield

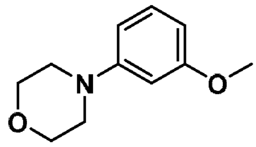

**3l**
93% yield

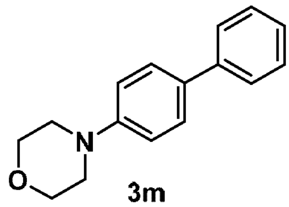

**3m**
65% yield

# Fig. 2

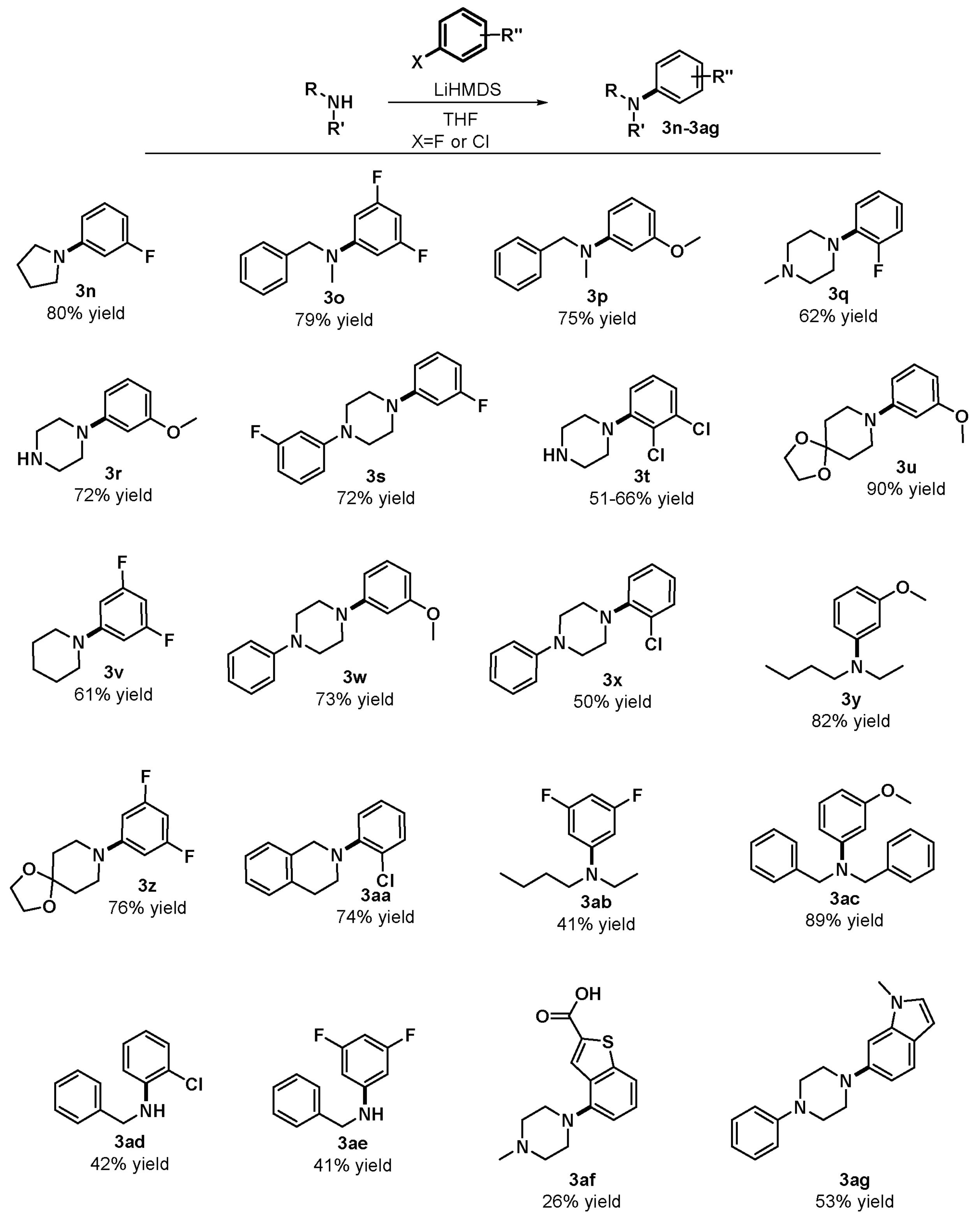


3n
80% yield

3o
79% yield

3p
75% yield

3q
62% yield

3r
72% yield

3s
72% yield

3t
51-66% yield

3u
90% yield

3v
61% yield

3w
73% yield

3x
50% yield

3y
82% yield

3z
76% yield

3aa
74% yield

3ab
41% yield

3ac
89% yield

3ad
42% yield

3ae
41% yield

3af
26% yield

3ag
53% yield

**Fig. 3**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014191548 A


### Non-patent literature cited in the description


- Without electron-attracting groups present, nucleophilic aromatic substitution occurs only under extreme reaction conditions. **F. A. CAREY ; R. J.** Sundberg in Advanced Organic Chemistry: Part A: Structure and Mechanisms. Springer Science and Business Media, 2000
- To summarize: Any anion-stabilizing (electron-withdrawing) group ortho or para to a potential leaving group can be used to make nucleophilic aromatic substitution possible. **J. CLAYDEN ; N. GREEVES ; S. WARREN ; P. WOTHERS.** Organic Chemistry. University Press, 2001
- **LOUIE, J. et al.** Palladium-catalyzed Synthesis of Arylamines from Aryl Halides. Mechanistic Studies Lead to Coupling in the Absence of Tin Reagents. *Tetrahedron Letters,* 1995, vol. 36 (21), 3609-3612
- **DINESS, F. et al.** Catalyst-Free N-Arylation Using Unactivated Fluorobenzenes. *Angewandte Chemie Int. Ed.,* 2012, vol. 51 (32), 8012-8016
- **BORDWELL.** *Acc. Chem. Res.,* 1988, vol. 21, 456-463
- **HANSCH et al.** *Chem. Rev.,* 1991, vol. 91, 165-195
- **C. DESMARETS ; R. SCHNEIDER ; Y. FORT.** *J. Org. Chem.,* 2002, vol. 67, 3029-3036
- **N. KATAOKA ; Q. SHELBY ; J. P. STAMBULI ; J. F. HARTWIG.** *J. Org. Chem.,* 2002, vol. 67, 5553-5566
- **B. U. MAES ; K. T. LOONES ; S. HOSTYN ; G. DIELS ; G. ROMBOUTS.** *Tetrahedron,* 2004, vol. 60, 11559-11564
- **S. URGAONKAR ; J. G. VERKADE.** *J. Org. Chem.,* 2004, vol. 69, 9135-9142
- **I. C. LERMA ; M. J. CAWLEY ; F. G. CLOKE ; K. ARENTSEN ; J. S. SCOTT ; S. E. PEARSON ; J. HAYLER ; S. CADDICK.** *J. Organomet. Chem.,* 2005, vol. 690, 5841-5848
- **Q. SHEN ; T. OGATA ; J. F. HARTWIG.** *J. Am. Chem. Soc.,* 2008, vol. 130, 6586-6596
- **M. OTSUKA ; K. ENDO ; T. SHIBATA.** *Chem. Comm.,* 2010, vol. 46, 336-338
- **D. GUO ; H. HUANG ; Y. ZHOU ; J. XU ; H. JIANG ; K. CHEN ; H. LIU.** *Green Chem.,* 2010, vol. 12, 276-281
- **B. LÜ ; P. LI ; C. FU ; L. XUE ; Z. LIN ; S. MA.** *Adv. Synt. Cat.,* 2011, vol. 353, 100-112
- **JACOBSEN, C. B. ; MELDAL, M. ; DINESS, F.** *Chemistry - A European Journal,* 2016